(19) Europäisches Patentamt
European Patent Office
Office européen des brevets

(11) EP 2 902 503 A1

# (12) EUROPEAN PATENT APPLICATION

(43) Date of publication:
**05.08.2015 Bulletin 2015/32**

(51) Int Cl.:
***C12Q 1/68*** *(2006.01)*

(21) Application number: **14382033.0**

(22) Date of filing: **31.01.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
- **Fundación Imdea Nanociencia**
  **28049 Madrid (ES)**
- **The Regents of the University of California**
  **Oakland, CA 94607 (US)**

(72) Inventors:
- **Somoza Calatrava, Álvaro**
  **E-28940 Madrid (ES)**
- **Latorre Lozano, Alfonso**
  **E-28490 Madrid (ES)**
- **Ortiz Urda, Susana**
  **Oakland, CA California 94607-5200 (US)**
- **Posch, Christian**
  **Oakland, CA California 94607-5200 (US)**

(74) Representative: **ABG Patentes, S.L.**
**Avenida de Burgos, 16D**
**Edificio Euromor**
**28036 Madrid (ES)**

## (54) Functionalized metal nanoparticles and uses thereof for detecting nucleic acids

(57) The present invention relates to metal nanoparticles functionalized with an oligonucleotide, to a method for the preparation thereof, to methods for detecting the presence of a nucleic acid in a sample or a target sequence in said nucleic acid using said nanoparticles, as well as to kits comprising the reagents necessary for obtaining said nanoparticles.

EP 2 902 503 A1

SEQUENCE LISTING

<110> Fundación IMDEA Nanociencia
The Reagents of the University of California

<120> FUNCTIONALIZED METAL NANOPARTICLES AND USES THEREOF FOR DETECTING NUCLEIC ACIDS

<130> P10179EP00

<160> 13

<170> PatentIn version 3.5

<210> 1
<211> 32
<212> DNA
<213> Artificial Sequence

<220>
<223> KRAS-WT-Cole

<400> 1
agcaagacgc caccagctcc acttgctaaa aa 32

<210> 2
<211> 32
<212> DNA
<213> Artificial Sequence

<220>
<223> KRAS-MUT-Cole

<400> 2
agcaagacgc cacaagctcc acttgctaaa aa 32

<210> 3
<211> 15
<212> DNA
<213> Artificial Sequence

<220>
<223> KRAS-WT-Target

<400> 3
tggagctggt ggcgt 15

<210> 4
<211> 15
<212> DNA
<213> Artificial Sequence

<220>
<223> KRAS-MUT-Target

<400> 4
tggagcttgt ggcgt 15

<210> 5

<211> 30
<212> DNA
<213> Artificial Sequence

<220>
<223> GNAQ-WT-Cole

<400> 5
ccgtctgacc tttggccccc gacggaaaaa 30

<210> 6
<211> 30
<212> DNA
<213> Artificial Sequence

<220>
<223> GNAQ-MUT-Cole

<400> 6
ccgtctgacc ttgggccccc gacggaaaaa 30

<210> 7
<211> 15
<212> DNA
<213> Artificial Sequence

<220>
<223> GNAQ-WT-Target

<400> 7
gggggccaaa ggtca 15

<210> 8
<211> 15
<212> DNA
<213> Artificial Sequence

<220>
<223> GNAQ-MUT-Target

<400> 8
gggggcccaa ggtca 15

<210> 9
<211> 28
<212> DNA
<213> Artificial Sequence

<220>
<223> RP-WT-Cole

<400> 9
ctccccgtgg cgccagttgg gagaaaaa 28

<210> 10
<211> 28
<212> DNA
<213> Artificial Sequence

```
<220>
<223>  RP-MUT-Cole

<400>  10
ctccccgtgg caccagttgg gagaaaaa                                        28


<210>  11
<211>  15
<212>  DNA
<213>  Artificial Sequence

<220>
<223>  RP-WT-Target

<400>  11
caactggcgc cacgg                                                      15


<210>  12
<211>  15
<212>  DNA
<213>  Artificial Sequence

<220>
<223>  RP-MUT-Target

<400>  12
caactggtgc cacgg                                                      15


<210>  13
<211>  28
<212>  DNA
<213>  Artificial Sequence

<220>
<223>  RP-MUT-Control

<400>  13
ctcccaactg gtgccacggg gagaaaaa                                        28
```

## Description

Field of the Invention

The present invention relates to metal nanoparticles functionalized with an oligonucleotide, to a method for the preparation thereof, to methods for detecting the presence of a nucleic acid in a sample or a target sequence in said nucleic acid using said nanoparticles, as well as to kits comprising the reagents necessary for obtaining said nanoparticles.

Background of the Invention

The use of gold nanoparticles has many applications in biology and medicine as mechanisms useful for transporting substances of interest, for example drugs. The use of metal nanoparticles as sensors stands out among other applications. In most cases, the use of gold nanoparticles as sensors is based on the change in color occurring in the solution in which the nanoparticles are found because when gold nanoparticles are in a colloidal dispersion, the color of the solution is reddish, whereas when said nanoparticles aggregate, the color of the solution becomes bluish.

Other sensors are based on the properties of fluorescence quenching of gold nanoparticles. Gold nanoparticles functionalized with molecular beacons coupled to a fluorophore have been described for the detection of nucleic acids based on the shielding effect produced by the nanoparticles, such that fluorescence is detected only when a conformational change occurs due to hybridization between the probe and the nucleic acid of interest (Y. Tu, et al., 2012, Chem. Commun., 48: 10718-10720; WO 2002018951). Metal nanoparticles are known to interact with fluorophores close to their surface, affecting the intensity of their emission by either reducing it or increasing it. On the other hand, the distance between the fluorophore and the surface of the nanoparticle is critical. In this sense, it has been described that the fluorescence of a fluorophore is deactivated when the distance to the metal is too short. For this reason the sensitivity of the methods using these functionalized gold nanoparticles does not allow distinguishing nucleotide alterations in the sequence of the nucleic acid of interest. Furthermore, the usual preparation of such nanoparticles requires synthesizing oligonucleotides substituted with thiol by means of using commercial solid supports containing a protected thiol group. After synthesis and purification of the oligonucleotide, the protected thiol group has to be deprotected using an excess of a reagent such as DTT or phosphine derivatives. The use of said reducing agents can alter oligonucleotide stability, this process being critical particularly when the oligonucleotide is RNA.

Therefore, there is a need in the state of the art to provide nanoparticles functionalized with oligonucleotides that are more stable than those that are known today, as well as to develop a method which allows detecting alterations in a sequence of a nucleic acid of interest by means of using said nanoparticles.

Brief Description of the Invention

The authors of the present invention have developed metal nanoparticles functionalized with an oligonucleotide with a hairpin structure, comprising a hydrophobic molecule at the end opposite to which it is bound to the nanoparticle. The affinity between the hydrophobic group and the nanoparticle causes the hydrophobic group to be buried in the proximity of the nanoparticle. These nanoparticles are useful for the identification of nucleic acids specifically hybridizing with the oligonucleotide forming part the nanoparticles given that hybridization of the oligonucleotide with a nucleic acid results in the unfolding of the hairpin structure, with the subsequent exposure of the hydrophobic group to the exterior, and therefore in an alteration in the surface plasmon band of said nanoparticles. The plasmon band of the nanoparticles can be determined with high sensitivity, which allows distinguishing nanoparticle-nucleic acid complexes in which the nucleic acid shows 100% complementarity with the oligonucleotide forming part of the nanoparticle of complexes in which 100% complementarity with the nucleic acid does not exist. This allows using the nanoparticles of the invention both for the detection of the presence of a nucleic acid in a sample and for the identification of nucleic acids showing a certain mutation.

Therefore, in a first aspect the invention relates to a metal nanoparticle bound to an oligonucleotide, wherein said oligonucleotide comprises a first nucleotide region, a second nucleotide region and a third nucleotide region, wherein said first and third regions are at least partially complementary such that they can give rise to a hairpin-type structure by means of hybridization, and wherein said oligonucleotide is modified with a hydrophobic molecule and with a suitable group for the binding of the oligonucleotide to said metal nanoparticle at the end opposite to where the hydrophobic molecule is located.

In a second aspect, the invention relates to a method for obtaining the nanoparticle of the invention which comprises:

(i) contacting metal nanoparticles with an oligonucleotide, wherein said oligonucleotide comprises a first nucleotide region, a second nucleotide region and a third nucleotide region, wherein said first and third regions are at least

partially complementary such that they can give rise to a hairpin-type structure by means of hybridization, and wherein said oligonucleotide is modified with a hydrophobic molecule at one end and with a suitable group for the binding of the oligonucleotide to said metal nanoparticle at the opposite end; and
(ii) maintaining the nanoparticles under suitable conditions for the binding between said oligonucleotide and the metal of the nanoparticle.

In a third aspect, the invention relates to a method for detecting the presence of a nucleic acid in a sample which comprises:

(i) contacting a nanoparticle of the invention with said sample, wherein the second region of said oligonucleotide is capable of specifically hybridizing with the nucleic acid to be detected, wherein said contacting is carried out under conditions that allow hybridizing said region of the oligonucleotide of the nanoparticle with the nucleic acid of said sample; and
(ii) determining the surface plasmon band intensity of the nanoparticles,

wherein a decrease in the surface plasmon band intensity of the nanoparticles of the sample with respect to the surface plasmon band intensity of the nanoparticles in the absence of the nucleic acid is indicative of the presence of said nucleic acid in said sample.
In a fourth aspect, the invention relates to a method for detecting the presence of a target sequence in a nucleic acid which comprises:

(i) separately contacting said nucleic acid with a first type of nanoparticles according to the invention and with a second type of nanoparticles according to the invention, wherein the oligonucleotide of said first type of nanoparticles contains a second region that is at least partially complementary with said target sequence, and wherein the oligonucleotide of said second type of nanoparticles is capable of hybridizing with the target sequence with less affinity than the second region of the oligonucleotide of the first type of nanoparticles, and wherein said contacting is carried out under conditions that allow hybridizing said second regions of the oligonucleotides of the first type and of the second type of nanoparticles with the nucleic acid; and
(ii) determining the surface plasmon band intensity of said first and second types of nanoparticles in the presence of the nucleic acid

wherein a decrease in the surface plasmon band intensity of the first type of nanoparticles with respect to the surface plasmon band intensity of said second type of nanoparticles obtained in step (ii) is indicative of the presence of said target sequence in the nucleic acid.
In a fifth aspect, the invention relates to the use of the nanoparticle of the invention for detecting the presence of a nucleic acid in a sample or for detecting a target sequence in a nucleic acid.
Finally, the invention also relates to kits comprising the nanoparticles of the invention, as well as to kits containing the suitable reagents for obtaining the nanoparticles of the invention from metal nanoparticles and from modified oligonucleotides.

## Brief Description of the Drawings

Figure 1. Electron transmission microscopy images of the nanoparticles modified with oligonucleotides.
Figure 2. UV-VIS spectrum of gold nanoparticles designed for detecting the non-mutated KRAS sequence in the presence of the target sequence (Match), mutated KRAS sequence (Mismatch) and a random sequence (SCR) at a concentration of 32 nM. A pronounced drop in absorbance in the presence of the target sequence is observed.
Figure 3. UV-VIS spectrum of gold nanoparticles designed for detecting the mutation of the KRAS sequence in the presence of the target sequence (Match), non-mutated KRAS sequence (Mismatch) and a random sequence (SCR) at a concentration of 32 nM. A pronounced drop in absorbance in the presence of the target sequence is observed.
Figure 4. UV-VIS spectrum of gold nanoparticles designed for detecting the non-mutated GNAQ sequence in the presence of the target sequence (Match), mutated GNAQ sequence (Mismatch) and a random sequence (SCR) at a concentration of 3.2 nM. A pronounced drop in absorbance in the presence of the target sequence is observed.
Figure 5. UV-VIS spectrum of gold nanoparticles designed for detecting the mutated GNAQ sequence in the presence of the target sequence (Match), non-mutated GNAQ sequence (Mismatch) and a random sequence (SCR) at a concentration of 3.2 nM. A pronounced drop in absorbance in the presence of the target sequence is observed.
Figure 6. UV-VIS spectrum of gold nanoparticles designed for detecting the non-mutated PDE6B sequence in the

presence of the target sequence (Match), mutated PDE6B sequence (Mismatch) and a random sequence (SCR) at a concentration of 3.2 nM. A pronounced drop in absorbance in the presence of the target sequence is observed.

Figure 7. UV-VIS spectrum of gold nanoparticles designed for detecting the mutated PDE6B sequence in the presence of the target sequence (Match), non-mutated PDE6B sequence (Mismatch) and a random sequence (SCR) at a concentration of 3.2 nM.

Figure 8. UV-VIS spectrum of gold nanoparticles without cholesterol designed for detecting the mutated PDE6B sequence in the presence of the target sequence (Match), non-mutated PDE6B sequence (Mismatch) and a random sequence (SCR) at a concentration of 3.2 nM. No drop in the absorbance in the presence of any of the target sequences is observed. The importance of the presence of the cholesterol molecule for sensor effectiveness is proven.

Figure 9. UV-VIS spectrum of gold nanoparticles designed for detecting the non-mutated GNAQ gene in the presence of RNA extracts (5.5 ng/μL) obtained from C918 cells, OMM1.3 cells and mouse liver. A pronounced drop in absorbance in the presence of extracts having the GNAQ gene is observed.

Figure 10. UV-VIS spectrum of gold nanoparticles designed for detecting the non-mutated GNAQ gene in the presence of RNA extracts (5.5 ng/μL) obtained from OMM1.3 cells, C918 cells and mouse liver. A pronounced drop in absorbance in the presence of the extract containing the mutated GNAQ gene is observed.

Figure 11. UV-VIS spectrum of gold nanoparticles designed for detecting the mutated PDE6B gene in the presence of RNA extracts (5.5 ng/μL) obtained from mouse liver, MEL202 cells and SKMEL2 cells. A pronounced drop in absorbance in the presence of the extract containing the mutated RP gene is observed.

## Detailed Description of the Invention

### Nanoparticle of the invention

In a first aspect, the invention relates to a metal nanoparticle bound to an oligonucleotide, wherein said oligonucleotide comprises a first nucleotide region, a second nucleotide region and a third nucleotide region, wherein said first and third regions are at least partially complementary such that they can give rise to a hairpin-type structure by means of hybridization, and wherein said oligonucleotide is modified with a hydrophobic molecule at one end and with a suitable group for the binding of the oligonucleotide to said metal nanoparticle at the end opposite to where the hydrophobic molecule is located.

The term "nanoparticle" is used to designate colloidal systems of the spherical type, rod type, polyhedron type, etc., or similar shapes, having a size less than 1 micrometer (μm), which are individually found or are found forming organized structures (dimers, trimers, tetrahedrons, etc.), dispersed in a fluid (aqueous solution). In a particular embodiment, the nanoparticles suitable for putting the invention into practice have a size less than 1 μm, generally comprised between 1 and 999 nanometers (nm), typically between 5 and 500 nm, preferably between about 10 and 150 nm. In a particular embodiment, the nanoparticles of the invention typically have a mean particle diameter ranging from 2 to 50 nm, preferably from 5 to 20 nm, more preferably of 13 nm. The mean particle diameter is the maximum mean particle dimension, with the understanding that the particles are not necessarily spherical. The shape of said nanoparticles can widely vary; advantageously, said nanoparticles will adopt any optically efficient shape such as spheres, rods, stars, cubes, polyhedrons or any other variant as well as complex associations of several particles; in a particular embodiment, the shape of the nanoparticles for putting the invention into practice is spherical or substantially spherical. The shape can be suitably evaluated by conventional light or by means of electron microscopy techniques.

The term "metal nanoparticle" refers to a nanoparticle comprising a metal and showing the optical property known as the surface plasmon phenomenon, i.e., a plasmonic metal. This phenomenon consists of the collective vibration of the electrons of the metal surface, producing an absorption band located in the ultraviolet-visible spectrum (typical of the metal and of the size of the nanoparticles) at the wavelength where the resonance condition occurs in said electrons. The surface plasmon of a metal can be determined by means of any spectroscopic technique known in the state of the art, such as surface plasmon resonance (SPR) spectroscopy, whereby the metal atoms are subjected to an electromagnetic beam or surface plasmon resonance fluorescence spectroscopy (SPFS) based on the detection of the variation of the refractive index of the metal atoms when they are subjected to a photon beam. As defined herein, a "plasmonic metal" is a metal characterized by showing the property of optics known as the surface plasmon phenomenon. The variation of the plasmonic response is particularly evident when several nanoparticles are located close to one another, given that this causes the coupling of their respective near fields, generating a new surface plasmon. In a particular embodiment, said metal is selected from the group consisting of gold, silver, copper, aluminum, platinum, iron, cobalt, palladium and combinations thereof. In an even more particular embodiment, the metal forming said nanoparticle is gold.

The nanoparticle according to the present invention is bound to an oligonucleotide. As it is used herein, the term "oligonucleotide" refers to a nucleic acid preferably of at least 10 nucleotides, preferably at least 16 nucleotides, preferably at least 20 nucleotides, preferably at least 25 nucleotides, and preferably not more than 100 nucleotides,

including both polyribonucleotides and polydeoxyribonucleotides or combinations thereof. The term "oligonucleotide" also refers to molecules formed by conventional nucleotides bound by conventional phosphodiester bonds as well as variants thereof, including modifications in the purine or pyrimidine residues and modifications in the ribose or deoxyribose residues designed for increasing biological stability of the oligonucleotide or for stabilizing the physical stability of the hairpin-shaped structure. Examples of modified nucleotides that can be used in the present invention include, but are not limited to, nucleotides having at position 2' of the sugar a substituent selected from the fluoro, hydroxyl, amino, azido, alkyl, alkoxy, alkoxyalkyl, methyl, ethyl, propyl, butyl group or a functionalized alkyl group such as ethylamino, propylamino and butylamino. Alternatively, the alkoxy group is methoxy, ethoxy, propoxy or a functionalized alkoxy group according to the formula $-O(CH_2)q-R$, where q is 2 to 4 and R is an amino, methoxy or ethoxy group. Suitable alkoxyalkyl groups are methoxyethyl and ethoxyethyl. Oligonucleotides in which different nucleotides contain different modifications at position 2' are also part of the invention. Alternatively or additionally, the oligonucleotides of the invention can contain modified bonds such as phosphodiester-, phosphotriester-, phosphorothioate-, phosphorodithioate-, phosphoroselenoate-, phosphorodiselenoate-, phosphoroanilothioate-, phosphoramidate-, methylphosphonate-, boranephosphonate-type bonds as well as combinations thereof, or they are peptide nucleic acids (PNAs), in which the different nucleotides are bound by amide bonds. The chemical synthesis of nucleic acids is a well-known technique and it can be performed, for example, by means of using an automatic DNA synthesizer (such as commercially available Bioautomation synthesizers, for example). Said oligonucleotide can be synthesized by methods known in the art as the solid-phase phosphoramidite method, the triester method or the phosphorothioate method. For example, said oligonucleotide can be synthesized on a solid support using a MerMade (Bioautomation) synthesizer, as shown in Example 3 of the present application. Other conventional solid supports for oligonucleotide synthesis that are well known by the person skilled in the art include, but are not limited to, inorganic substances such as silica, porous glass, aluminosilicates, borosilicates, metal oxides, etc. Alternatively, said solid support can be an organic substance, such as a cross-linked polymer, such as polyamide, polyether, polystyrene, etc. The preferred solid support for use in the synthesis of the oligonucleotide forming the nanoparticle according to the present invention is controlled porous glass (CPG).

The oligonucleotide comprises a first nucleotide region, a second nucleotide region and a third nucleotide region, in that order, wherein said first and third regions are at least partially complementary such that they can give rise to a hairpin-type structure by means of hybridization.

As it is used herein, the term "partially complementary" refers to said regions having a degree of complementarity that is sufficient for specifically hybridizing. Absolute complementarity between said first and third regions of the oligonucleotide is not required although it is preferred. In a preferred embodiment, said degree of complementarity between said first and third regions of said oligonucleotide is at least 10%, more preferably said degree of complementarity is at least 20%, more preferably said degree of complementarity is at least 30%, more preferably said degree of complementarity is at least 40%, more preferably said degree of complementarity is at least 50%, more preferably said degree of complementarity is at least 60%, more preferably said degree of complementarity is at least 70%, more preferably said degree of complementarity is at least 80%, more preferably said degree of complementarity is at least 90%, more preferably said degree of complementarity is at least 95%, more preferably said degree of complementarity is at least 99%, even more preferably said degree of complementarity between said first and third regions is 100%. The degree of complementarity between both regions is determined by means of comparing the order of the nitrogen bases forming said first and third regions without taking into account structural differences between said first and third regions, provided that said structural differences do not prevent the formation of hydrogen bridges between the complementary bases. The degree of complementarity between said first and third regions can also be determined in terms of the number of unpaired bases present between said first and third regions. As the person skilled in the art will understand, the hybridization capacity between said first and third regions will depend on the degree of complementarity between both regions and on the length of the oligonucleotide.

In a preferred embodiment, said first and third regions of said oligonucleotide forming the nanoparticle of the invention are of equal length, i.e., they comprise the same number of nucleotides. In another preferred embodiment, said first and third regions are of different lengths, wherein the first region comprises more nucleotides than the third region, or wherein the third region comprises more nucleotides than the first region. Said first region typically comprises 2, 3, 4, 5, 6, 7, 8, 9, 10, 11, 12, 13, 14, 15, 20, 25, 30, 40 or 50 nucleotides. Said third region typically comprises 2, 3, 4, 5, 6, 7, 8, 9, 10, 11, 12, 13, 14, 15, 20, 25, 30, 40 or 50 nucleotides.

Said oligonucleotide forming the nanoparticle of the invention comprises a second nucleotide region flanked by said first and third nucleotide regions. In a preferred embodiment, said second region comprises 5, 6, 7, 8, 9, 10, 11,12, 13, 14, 15, 20, 25, 30, 40, 50, 60, 70, 80, 90, 95 or 100 nucleotides. In a particular embodiment of the invention, said second region comprises a nucleotide sequence complementary to a target sequence. In a preferred embodiment, said target sequence is selected from the group consisting of the GNAQ gene, the PDE6B gene, and the KRAS gene. Even more preferred embodiments of the invention contemplate nanoparticles covalently bound to an oligonucleotide, wherein said oligonucleotide comprises a sequence selected from the sequences shown in SEQ ID NO: 1, SEQ ID NO: 2, SEQ ID NO: 5, SEQ ID NO: 6, SEQ ID NO: 9 or SEQ ID NO: 10.

Hybridization between the first and third regions of said oligonucleotide forming the nanoparticle of the invention gives rise to the formation of a hairpin-type structure. As it is used herein, a "hairpin-type structure" or "hairpin loop" refers to a secondary structure formed by an oligonucleotide comprising a stem region and a loop region, wherein said stem region is formed by a sequence of a double-stranded oligonucleotide formed by the hybridization between two or more nucleotide regions which are partially complementary to one another, and wherein said loop region is formed by a single-stranded sequence comprising unpaired bases. In the present invention, hybridization between the first and third regions of the oligonucleotide forming the nanoparticle of the invention gives rise to said stem region, and the second region of said oligonucleotide gives rise to the loop region of the hairpin-type structure. In a preferred embodiment, the stem region comprises from 2 to 50 paired nucleotides, more preferably 3 paired nucleotides, 4 paired nucleotides, 5 paired nucleotides, 6 paired nucleotides, 7 paired nucleotides, 8 paired nucleotides, 9 paired nucleotides, 10 paired nucleotides, 15 paired nucleotides, 20 paired nucleotides, 30 paired nucleotides, 40 paired nucleotides or 50 paired nucleotides; and the loop region preferably comprises 3 nucleotides, 4 nucleotides, 5 nucleotides, 6 nucleotides, 7 nucleotides, 8 nucleotides, 9 nucleotides, 10 nucleotides, 15 nucleotides, 20 nucleotides, 30 nucleotides, 40 nucleotides, 50 nucleotides or more.

The oligonucleotide forming part of the metal nanoparticle of the invention has modifications at the 3' and 5' ends. At one of its ends, the oligonucleotide is modified with a suitable group for the binding of the oligonucleotide to the metal nanoparticle. As it is used herein, the term "suitable group for the binding of the oligonucleotide to the metal nanoparticle" refers to a group capable of reacting with the metal atoms of the nanoparticle allowing the binding between the oligonucleotide and the nanoparticle. In a particular embodiment, said suitable group for the binding of the oligonucleotide to the metal nanoparticle is selected from a group containing a sulfur atom and a polyadenine sequence.

In a particular embodiment, said oligonucleotide forming the nanoparticle of the invention is modified with a group containing a sulfur atom at one end and with a hydrophobic molecule at the opposite end. The term "group containing a sulfur atom" refers to a set of atoms containing sulfur which are bound to a carbon chain by means of a covalent bond. The group containing sulfur is preferably associated with the oligonucleotide in the same way that the different nucleotides are associated with one another, i.e., through oxygen at position 3' or at position 5' of the sugar (ribose or deoxyribose) ring. In a particular and preferred embodiment of the invention, the group containing a sulfur atom is bound to the nucleotide located at the 3' end of the oligonucleotide. In that case, the group containing sulfur is bound to the oxygen at position 5' of the ribose or deoxyribose ring. In another particular embodiment, the group containing the sulfur atom is bound to the 5' end of the oligonucleotide. In that case, the group containing sulfur is bound to the oxygen at position 3' of the ribose or deoxyribose ring. Methods for obtaining oligonucleotides modified with groups containing sulfur are known in the state of the art and described in the second aspect of the invention.

In a particular embodiment, the binding between the oligonucleotide and the nanoparticle in the metal nanoparticle of the invention takes place through sulfur-metal bond formed between the metal atoms located on the surface of the nanoparticle and the sulfur group which is bound to the oligonucleotide. In a preferred embodiment, the sulfur-metal bond is formed by reaction between the metal atoms of the nanoparticle and a sulfur atom forming part of a functional group containing sulfur.

In a preferred embodiment, the sulfur-metal bond is a covalent bond. The terms "covalent", "covalent binding", "covalently bound" must be understood as the formation of a bond between two atoms or groups of atoms by sharing electron pairs.

In a particular embodiment, the covalent sulfur-metal bond is formed by means of a reaction between a sulfur atom of a functional group containing sulfur and a metal atom of the nanoparticle. The term "functional group containing sulfur" refers to a set of atoms containing sulfur which are bound to a carbon chain by means of a covalent bond which confer reactivity to the molecule containing them. In a particular embodiment, said functional group containing sulfur is selected from a thiol group, a dithiolane group and an isocyanide group.

As it is used herein, "thiol group" refers to a molecule formed by a sulfur atom and a hydrogen atom (SH). As it is used herein, an "amine group" refers to an organic chemical compound derived from ammonia in which one or several hydrogens of the ammonia molecule are substituted with other substituents or radicals. As it is used herein, an "isocyanide group" is an organic compound with the functional group R-N≡C. As it is used herein, "a dithiolane group" refers to a heterocyclic compound derived from cyclopentane containing sulfur formed by means of substituting two methyl groups ($CH_2$) with two thioether groups. The substituted methyl groups can be groups located at contiguous positions or at alternating positions in the heterocyclic ring. If two thioether groups substitute two methyl groups located at contiguous positions, then 1,2-dithiolane is obtained. If two thioether groups substitute two methyl groups occupying alternating positions in the heterocyclic molecule, then 1,3-dithiolane is obtained. In a preferred embodiment of the invention, said functional group containing sulfur is 1,2-dithiolane.

In a more particular embodiment, the group containing the sulfur atom is bound to the oligonucleotide through a spacer sequence. The spacer sequence can be located at the 5' end of the oligonucleotide in the event that the group containing sulfur is located at the 5' end of the oligonucleotide, or it may be located at the 3' end of the oligonucleotide in the event that the group containing sulfur is located at the 3' end of the oligonucleotide. As it is used herein, "spacer

sequence" refers to a nucleotide sequence with which binding with a molecule of interest is carried out, particularly with said group containing the sulfur atom. In a particular embodiment of the invention, said binding sequence is a polyadenine sequence. In a preferred embodiment of the invention, the binding between said oligonucleotide and the group containing a sulfur atom is performed through a binding sequence formed by 5 adenines.

In another particular embodiment, said oligonucleotide forming the metal nanoparticle of the invention is modified with a polyadenine sequence at the end opposite the end where it is modified with said hydrophobic molecule. Polyadenine sequences are capable of self-assembling by means of adsorption on the surface of metal particles. In a preferred embodiment, said polyadenine sequence is formed by at least 5 adenines; in another preferred embodiment, said polyadenine sequence is formed by at least 10 adenines, at least 15 adenines, at least 20 adenines, at least 25 adenines, at least 30 adenines, at least 35 adenines, at least 40 adenines, at least 45 adenines, at least 50 adenines or more. The polyadenine sequence is preferably associated with the oligonucleotide in the same way that the different nucleotides are associated with one another, i.e., through the oxygen at position 3' or at position 5' of the sugar (ribose or deoxyribose) ring. In a particular and preferred embodiment of the invention, the polyadenine sequence is bound to the nucleotide located at the 3' end of the oligonucleotide. In another particular embodiment, the group the polyadenine sequence is bound to the 5' end of the oligonucleotide. The binding between said polyadenine sequence and the oligonucleotide forming the nanoparticle of the invention takes place through a phosphodiester bond. Methods for obtaining oligonucleotides modified with a polyadenine sequence are known in the state of the art and described in the second aspect of the invention.

In a particular embodiment, the binding between the polyadenine sequence and the nanoparticle is produced by adsorption of said polyadenine sequence on the metal atoms of the nanoparticle. As it is used herein, the term "adsorption" refers to the process whereby atoms, ions or molecules are trapped or retained on the surface of a material.

The oligonucleotide forming the nanoparticle of the invention is modified with a hydrophobic molecule at the end opposite the end where it is modified with a group containing a sulfur atom or with a polyadenine sequence. The term "oligonucleotide modified with a hydrophobic molecule" refers to an oligonucleotide comprising, in addition to the nucleotides forming it, at least one hydrophobic molecule bound to said oligonucleotide, preferably through one of the hydroxyl groups of the sugar molecules forming the nucleotides. As it is used herein, the term "hydrophobic molecule" refers to a molecule that is more soluble in organic solvents (for example, long-chain alcohols, esters, ethers, ketones and hydrocarbons) than in aqueous solutions. Basically, hydrophobicity occurs when the molecule referred to as hydrophobic is not capable of interacting with water molecules through ion-dipole interactions or by means of hydrogen bridges. Hydrophobicity of the "hydrophobic molecule" can be defined by the concentration ratio (partition coefficient) of the molecule distributed between an aqueous phase and an organic phase. Specifically, hydrophobicity can be defined by the partition coefficient log value (partition coefficient P (logP)) between the aqueous and organic phases (for definitions of the terms partition coefficient and partition ratio, refer to "Handbook of Chemistry, 5th ed., Basic II" [The Chemical Society of Japan ed., MARUZEN Co., Ltd., p. 168-177]). In the present invention, "logP" refers to a theoretical distribution coefficient calculated using a logP calculation program (for example, ACD/LogP, (version 9.0)), available from Advanced Chemistry Development, Inc., and an algorithm described in the ACD/LogP manual (2005) included therein. In the present invention, a "hydrophobic molecule" is defined as an organic molecule having a logP of 2 or more, preferably a logP of 7 or more, at pH 7. Specifically, the compound having a logP of 2 or more (i.e., the concentration ratio of the compound between the aqueous and organic phases is 1:100 or more) can be interpreted as having the property of forming aggregates through hydrophobic interactions with other molecules when they are distributed in a neutral aqueous solution.

The person skilled in the art will understand that the nanoparticles of the present invention are not limited in terms of the number of hydrophobic molecules modifying the oligonucleotide. Thus, it is possible to introduce more than one hydrophobic group in the oligonucleotide forming part of the nanoparticle. Thus, in preferred embodiments, the oligonucleotide is modified with two, three, four, five, six, seven, eight, nine, ten or more hydrophobic groups.

In a preferred embodiment, two or more nucleotides that are located at the 5' end of said oligonucleotide are modified with a hydrophobic molecule. In a preferred embodiment, three, four, five, six, seven, eight, nine, ten, fifteen or more nucleotides that are located at positions prior to the 5' end of the oligonucleotide are modified with a hydrophobic group.

In another preferred embodiment, the oligonucleotide of the 5' end is modified with two, three, four, five, six, seven, eight, nine, ten or more hydrophobic groups. In another preferred embodiment, three, four, five, six, seven, eight, nine, ten, fifteen or more nucleotides that are located at positions prior to the 5' end of the oligonucleotide are modified with two, two, three, four, five, six, seven, eight, nine, ten or more hydrophobic groups.

In a particular embodiment, said hydrophobic molecule is a lipid. As it is used herein, "lipid" refers to organic molecules that have a long-chain fatty acid or a hydrocarbon chain and are slightly soluble in water and readily soluble in organic solvents. In a more particular embodiment, said lipid is a steroid lipid. As it is used herein, "steroid lipid" refers to lipids derived from the nucleus of the hydrocarbon sterane (or cyclopentaneperhydrophenanthrene), being made up of four rings fused with functional hydroxyl and carbonyl groups. In a more particular embodiment, said steroid lipid is

cholesterol. As it is used herein, "cholesterol" refers to a sterane molecule having two methyl radicals at positions $C_{10}$ and $C_{13}$, a branched aliphatic chain containing eight carbons at position $C_{17}$, a hydroxyl group at position $C_3$ and an unsaturation between the $C_5$ and $C_6$ carbons; where C refers to a carbon atom, and wherein the number represented next to said C refers to the specific position said carbon atom occupies in the molecule.

In a particular and preferred embodiment of the invention, the group containing sulfur is bound to the 3' end of the oligonucleotide, in which case the hydrophobic molecule is bound to the 5' end of the oligonucleotide. In another particular embodiment, the group containing sulfur is bound to the 5' end of the oligonucleotide, in which case the hydrophobic molecule is bound to the 3' end of the oligonucleotide. In another particular embodiment, the polyadenine sequence is bound to the 3' end of the oligonucleotide, in which case the hydrophobic molecule is bound to the 5' end of the oligonucleotide. In another particular embodiment, the polyadenine sequence is bound to the 5' end, in which case the hydrophobic molecule is bound to the 3' end of the oligonucleotide. Methods for binding a hydrophobic molecule to an end of an oligonucleotide are well known in the state of the art and described in the context of the second aspect of the invention. The incorporation of the hydrophobic group into the oligonucleotide is preferably carried out by means of using precursors of said groups activated with a phosphoramidite group, which allows the incorporation of the hydrophobic group by reacting the phosphoramidite group with a hydroxyl group in the sugar molecule of the oligonucleotide. In a preferred embodiment, the incorporation of the hydrophobic group is carried out by means of using the precursor cholesteryl-3-carboxamidohexyl-[(2-cyanoethyl)-(N,N-diisopropyl)]-phosphoramidite.

The nanoparticle of the invention has amphipathic characteristics, i.e., it has a water-soluble, hydrophilic region in its structure formed by the oligonucleotide forming part of said nanoparticle, and a hydrophobic region formed by the hydrophobic molecule bound to an end of the oligonucleotide. The amphipathicity of the molecule is variable depending on the folding state of the hairpin-shaped region. Thus, if the region with the hairpin structure is folded, the nanoparticle is more hydrophilic given that the loop of said region, which is hydrophilic, is exposed to the exterior. If the region with the hairpin structure is unfolded, the nanoparticle is more hydrophobic given that the hydrophobic group is exposed to the exterior of the nanoparticle.

<u>Process for obtaining the nanoparticle of the invention</u>

In a second aspect, the invention relates to a process for obtaining the nanoparticle of the invention which comprises:

(i) contacting metal nanoparticles with an oligonucleotide, wherein said oligonucleotide comprises: a first nucleotide region, a second nucleotide region and a third nucleotide region, wherein said first and third regions are at least partially complementary such that they can give rise to a hairpin-type structure by means of hybridization, and wherein said oligonucleotide is modified with a hydrophobic molecule at one end and with a suitable group for the binding of the oligonucleotide to said metal nanoparticle at the end opposite to where the hydrophobic molecule is located; and

(ii) maintaining the nanoparticles under suitable conditions for the binding between said oligonucleotide and the metal of the nanoparticle.

The terms "metal nanoparticle", "oligonucleotide", "suitable group for the binding of the oligonucleotide to the metal nanoparticle", "group containing sulfur", "polyadenine sequence", "hydrophobic molecule", "partially complementary" and their particular embodiments are defined in the first aspect of the invention.

The first step of the process for obtaining the metal nanoparticles of the invention comprises contacting metal nanoparticles with an oligonucleotide, wherein said oligonucleotide comprises a first nucleotide region, a second nucleotide region and a third nucleotide region, in that order, wherein said first and third regions are at least partially complementary such that they can give rise to a hairpin-type structure by means of hybridization, and wherein said oligonucleotide is modified with a hydrophobic molecule and with a suitable group for the binding of the oligonucleotide to said metal nanoparticle at the end opposite to where the hydrophobic molecule is located.

The metal nanoparticles used in the first step of said method can be obtained using any conventional methodology. The general method for obtaining metal nanoparticles is based on the preparation of an aqueous solution comprising one or more metal salts to which there is added a reducing agent capable of reducing the metal salt cation to the metal state.

In a particular embodiment of the invention, the method for obtaining the nanoparticles of the invention relates to obtaining gold nanoparticles. In a preferred embodiment of the invention, the metal salts used for obtaining said nanoparticles comprise gold salts, more preferably $HAuCl_4$. The reducing agents capable of reducing the metal salt cation to the metal state are known by the person skilled in the art (Bradley, J. S., Schmid, G., Talapin, D. V., Shevchenko, E. V. and Weller, H. (2005) Syntheses and Characterizations: 3.2 Synthesis of Metal Nanoparticles, in Nanoparticles: From Theory to Application (ed. G. Schmid), Wiley-VCH Verlag GmbH & Co. KGaA, Weinheim, FRG. doi:

10.1002/3527602399.ch3b. ISBN: 9783527305070). Hydrides, citrates, alcohols, reducing polymers, hydrazine, hydroxylamine or their derivatives and mixtures thereof can be used as reducing agents. In a preferred embodiment, the reducing agent used is trisodium citrate.

Once the metal nanoparticles in solution are obtained, they are collected, preferably by means of filtration or centrifugation, and optionally purified. The purification of the nanoparticles can be carried out by means of processes of washing/centrifuging in a suitable solvent, preferably water, or by means of dialysis. The nanoparticles thus obtained can be detected by UV-Vis spectroscopy, transmission electron microscopy (TEM) or Raman spectroscopy. In a preferred embodiment, the nanoparticles of the invention are detected by means of TEM and/or UV-Vis spectroscopy at 520 nm. The concentration of said nanoparticles can be estimated by means of any technique suitable for that purpose. By way of illustration, an estimation of the concentration of the obtained nanoparticles can be determined by means of the Lambert-Beer law or by means of determining the molar extinction coefficient, which is a parameter that defines the radiation absorbed by a substance at a certain wavelength per molar concentration.

If desired, the method for obtaining the nanoparticles of the invention can be carried out by means of the method of reduction with citrate illustrated in Example 6 of the application.

The first step of the method for obtaining the nanoparticles of the invention comprises contacting metal nanoparticles obtained by means of any suitable method with an oligonucleotide. Said oligonucleotide comprises a first nucleotide region, a second nucleotide region and a third nucleotide region, in that order, wherein said first and third regions are at least partially complementary such that they can give rise to a hairpin-type structure by means of hybridization, and wherein said oligonucleotide is modified with a hydrophobic molecule and with a suitable group for the binding of the oligonucleotide to said metal nanoparticle at the end opposite to where the hydrophobic molecule is located. In a particular embodiment, said suitable group for the binding of the oligonucleotide to the nanoparticle is selected from a functional group containing a sulfur atom and a polyadenine sequence.

In a particular embodiment, said oligonucleotide is modified with a functional group containing a sulfur atom at the end opposite to where it is modified with a hydrophobic molecule. In another particular embodiment, said oligonucleotide is modified with a polyadenine sequence at the end opposite to where it is modified with said hydrophobic molecule. Oligonucleotides modified with one or several functional groups can be obtained by means of any method known in the state of the art.

In a particular embodiment, the oligonucleotide forming the nanoparticle of the invention is modified at one of its ends with a functional group containing a sulfur atom selected from a thiol group, a dithiolane group, and an isocyanide group. In an even more particular embodiment of the invention, said functional group containing a sulfur atom is a dithiolane group. In a preferred embodiment of the invention, said functional group containing a sulfur atom is 1,2-dithiolane.

In a particular embodiment, the oligonucleotide forming the nanoparticle is modified at its 3' end with said functional group containing a sulfur atom. In another particular embodiment, the oligonucleotide forming part of the nanoparticle is modified at its 5' end with said functional group containing a sulfur atom.

The synthesis of an oligonucleotide modified at its 5' end or at its 3' end with a functional group containing a sulfur atom, for example with a dithiolane group, can be carried out by means of any suitable method known in the state of the art. Alternatively, the oligonucleotide can be acquired from a company (for example Sigma-Genosys), in which case it is important that the product is purified of other thiols, such as dithiothreitol (DTT) or dithioerythritol (DTE), which can be added as stabilizing agents. In such case, it is advisable to purify the oligonucleotide by means of any technique suitable for that purpose, such as for example, size exclusion chromatography, HPLC or electrophoresis in preparative gels.

If said oligonucleotide is synthesized using a DNA synthesizer, it can be carried out using the phosphoramidite method. Briefly, the synthesis method based on phosphoramidite chemistry is based on solid base synthesis of an oligonucleotide by means of the sequential addition of activated monomers. The activated monomers are protonated deoxyribonucleoside 3'-phosphoramidites. In a first step, the phosphorus atom at position 3' of the incoming element is bound to the oxygen atom at position 5' of the growing chain to form a phosphite triester. The hydroxyl group (OH) at position 5' of the activated monomer does not react because it is blocked by a dimethoxynitrile (DMT) protecting group, and the phosphoryl group at position 3' does not react because the beta-cyanoethyl group is bound thereto. Similarly, the amino groups of the purine and pyrimidine bases are blocked. Coupling is carried out under anhydrous conditions because the water reacts with phosphoramidites. In a second step, phosphite triester oxidizes to form a phosphate triester. In a third step, the DMT protecting group is detached from the OH at position 5' of the growing chain by means of adding a strong acid (such as trichloroacetic acid), leaving the protecting groups intact. At this point, the oligonucleotide has been elongated by one unit. The incorporation of the functional group containing sulfur can be carried out by means of adding to the synthesis reaction at the right time a monomer comprising a functional group with sulfur activated with a phosphoramidite group. The monomer with the functional sulfur group will thus be incorporated in the molecule.

In a particular embodiment, the oligonucleotide is synthesized with the functional group containing sulfur bound to its 3' end. Said oligonucleotide can be generated by starting the synthesis on a solid support (for example CPG)

modified with said group containing sulfur, such as a dithiolane group, for example, or alternatively by starting the synthesis on a conventional solid support and adding phosphoramidites modified with said dithiolane group as the first monomer in the sequence. Alternatively, the oligonucleotide can be synthesized with the dithiolane group bound to its 5' end. The synthesis of said oligonucleotide can be carried out by means of any technique suitable for that purpose, such as the phosphoramidite method, for example, whereby said nucleotide can be obtained by ending synthesis with phosphoramidites modified with a thiol group or a dithiolane group. In any case, as discussed above, it is advisable to perform a final treatment step under acidic conditions to release the hydroxyl group from the DTT group.

Example 2 of the present application illustratively shows obtaining said oligonucleotide by means of using a modified CPG support. Briefly, an oligonucleotide modified at the 3' end with a functional group containing a sulfur atom, for example a dithiolane group, can be obtained by means of a method of solid-phase synthesis comprising a first step wherein lipoic acid and threoninol are reacted to give rise to the corresponding amide derivative; then in a second step, the compound resulting from the first step is treated with succinic anhydride to introduce a carboxyl group, whereby causing the binding of the dithiolane group to CPG. Said modified support can be used as a reagent in the chemical synthesis of the oligonucleotide by means of using a conventional DNA synthesizer.

Oligonucleotides modified at 3' or 5' with a functional group containing sulfur, such as a thiol group or a dithiolane group, can be obtained using other conventional techniques based on the phosphodiester or phosphotriester methods (Narang et al., 1979, Meth. Enzymol., 68: 90; Brown et al., Meth. Enzymol., 1979, 68, 109).

In another particular embodiment, the oligonucleotide forming the nanoparticle of the invention is modified at one of its ends with a polyadenine sequence. In a particular embodiment, the oligonucleotide forming the nanoparticle is modified at its 3' end with said polyadenine sequence. In another particular embodiment, the oligonucleotide forming part of the nanoparticle is modified at its 5' end with said polyadenine sequence. The synthesis of an oligonucleotide modified at its 5' end or at its 3' end with a polyadenine sequence can be carried out by means of any suitable method known in the state of the art. Said modification can be carried out by means of chemical synthesis. Nucleic acid chemical synthesis is a well-known technique and can be performed by means of using an automatic DNA synthesizer, for example.

Said oligonucleotide is modified with a hydrophobic molecule at the end opposite to where it is modified with the functional group containing a sulfur atom. In a particular embodiment, the hydrophobic molecule is bound to the 5' end of the oligonucleotide. Alternatively, in another particular embodiment the invention contemplates said modification being performed at the 3' end of the oligonucleotide. Said oligonucleotide is modified with a hydrophobic molecule at the end opposite to where it is modified with a polyadenine sequence. In a particular embodiment, the hydrophobic molecule is bound to the 5' end of the oligonucleotide. Alternatively, in another particular embodiment the invention contemplates said modification being performed at the 3' end of the oligonucleotide.

In a particular embodiment, said hydrophobic molecule is a lipid. In a more particular embodiment, said hydrophobic molecule is a steroid lipid. In still a more particular embodiment, said steroid lipid is a cholesterol molecule.

Oligonucleotides modified at one end with a cholesterol molecule or with any other hydrophobic molecule can be obtained by means of conventional methods based on phosphoramidite chemistry. Alternatively, the chemical binding of the cholesterol molecule to the oligonucleotide can be carried out by means of other methods known in the state of the art, such as the methods based on the incorporation of lipids into oligonucleotides in the form of amino lipids (Chu et al., 1983, Nucl. Acids. Res. 11: 6513-6529).

In a preferred embodiment, the group containing the sulfur atom and the end of the oligonucleotide are bound through a spacer sequence. In an even more preferred embodiment, the spacer sequence is a polyadenine sequence.

In a preferred embodiment, said oligonucleotide comprises a sequence capable of hybridizing with a region of a gene selected from the group consisting of the GNAQ gene, the PDE6B gene, or the KRAS gene. In even more preferred embodiments of the invention, said oligonucleotide comprises a sequence selected from the sequences shown in SEQ ID NO: 1, SEQ ID NO: 2, SEQ ID NO: 5, SEQ ID NO: 6, SEQ ID NO: 9 or SEQ ID NO: 10.

Once said oligonucleotide is obtained by means of any suitable method, the second step of the process for obtaining the nanoparticles of the invention comprises maintaining the nanoparticles under suitable conditions for the binding between the oligonucleotide and the metal of the nanoparticle. As the person skilled in the art will understand, in said step the oligonucleotide may be added to the suspension containing the metal nanoparticles, or alternatively, a suitable solution could be prepared from a suspension containing metal nanoparticles and a suitable solvent. The oligonucleotide that is bound to the nanoparticle of the invention will then be added to said solution.

In a particular embodiment, said conditions will be suitable conditions for forming a bond between the sulfur atom of the functional group containing sulfur bound to an end of the oligonucleotide and the metal of the nanoparticle.

In a particular embodiment, the sulfur-metal bond is formed by reaction between a sulfur atom of the functional group containing sulfur of the oligonucleotide and a metal atom of the nanoparticle. In a preferred embodiment of the invention, said bond is formed between a sulfur atom of a dithiolane group bound to an end of the oligonucleotide and a gold atom of the nanoparticle. In a more preferred embodiment of the invention, said bond is formed between a sulfur atom of the dithiolane group bound to the 3' end of the oligonucleotide modified at the 5' end with a hydrophobic molecule, wherein said hydrophobic molecule is preferably a lipid, and more preferably cholesterol.

The binding between the oligonucleotide modified with a functional group containing a sulfur atom as described above and the metal atoms takes place spontaneously. Example 7 of the application shows suitable conditions for carrying out said conjugation. By way of illustration, said reaction can be carried out by incubating in a 0.3 M sodium chloride solution at a concentration of about 2.7 x $10^8$ $M^{-1}$ $cm^{-1}$ gold nanoparticles, obtained by any of the aforementioned methods, for example obtained by means of the citrate sodium method, with the modified oligonucleotide at a suitable concentration, preferably in excess, of about 10 μM for a suitable time interval so that the nanoparticles are coated by said oligonucleotide, for example for 16 hours. Said reaction is carried out under mild temperature and pH conditions, such as room temperature and physiological pH. After the reaction has occurred, the obtained nanoparticles are purified by means of three centrifugation cycles.

In another particular embodiment, the second step of the first method of the invention relates to suitable conditions so that the metal of the nanoparticle adsorbs the polyadenine sequence. The conjugation between the oligonucleotide modified with a polyadenine sequence and the metal nanoparticle can be carried out using conditions similar to those described for conjugating the metal nanoparticles with an oligonucleotide modified with a group containing a sulfur atom. <u>Method for detecting the presence of a nucleic acid in a sample</u>

The inventors have shown that the nanoparticles of the invention can be used for detecting nucleic acids in a sample which show sequence complementarity with the loop region of the oligonucleotides forming part of the nanoparticles of the invention. Detection is based on the change in the plasmon emission of the nanoparticle when the region with a hairpin structure unfolds as a result of hybridization between the loop region thereof and the nucleic acid that is in the sample, which gives rise to the exposure of the hydrophobic molecule to the exterior, which gives rise to an aggregation of nanoparticles and to a change (decrease) in the plasmon band of the nanoparticle.

Therefore, in a third aspect the invention relates to a method for detecting the presence of a nucleic acid in a sample which comprises:

(i) contacting a nanoparticle of the invention with said sample, wherein the second region of the oligonucleotide forming said nanoparticle is capable of specifically hybridizing with the nucleic acid to be detected, wherein said contacting is carried out under conditions that allow hybridizing said oligonucleotide of the nanoparticle with the nucleic acid of said sample; and

(ii) determining the plasmon band intensity of the nanoparticles

wherein a decrease in the plasmon band intensity of the nanoparticles of the sample with respect to the plasmon band intensity of the nanoparticles in the absence of the nucleic acid is indicative of the presence of said nucleic acid in said sample.

In the first step, the method for detecting the presence of a nucleic acid in a sample comprises contacting the nanoparticle of the invention with the sample of interest. In said step, the oligonucleotide forming part of the nanoparticle is capable of hybridizing with the nucleic acid to be detected. The nanoparticle of the invention and processes suitable for obtaining it are defined in the context of the first and second aspects of the present invention.

Said first step must take place under conditions that allow hybridizing said oligonucleotide with the target nucleic acid of the sample. As it is used herein, the term "hybridization" refers to the formation of a duplex-type structure by two nucleic acids due to complementary base pairing. Hybridization can occur between completely complementary nucleic acid chains or between "substantially complementary" nucleic acid chains containing small unpaired regions. The conditions with which completely complementary nucleic acid chains hybridize are referred to as "strict hybridization conditions" or "sequence-specific hybridization conditions" or "stringent conditions". Nevertheless, substantially complementary stable double strands of nucleic acids can be obtained under less strict or less stringent hybridization conditions, in which case, the tolerated degree of mismatch can be adjusted by means of suitable adjustment of the hybridization conditions. The person skilled in the art can empirically determine the stability of a duplex taking a number of variables into account, such as probe base pair length and concentration, ionic strength and mismatched base pair incidence, following the guidelines of the state of the art (see, for example, Sambrook et al., Molecular Cloning: A Laboratory Manual, Cold Spring Harbor Laboratory, Cold Spring Harbor, N.Y. (1989); and Wetmur, Critical Reviews in Biochem. and Mol. Biol. 26 (3/4):227-259 (1991)). In the context of the present invention, "specific hybridization" is understood as the binding, hybridizing or duplex-forming capacity of the second region of the oligonucleotide forming part of the nanoparticle of the invention with the target sequence to be detected such that said target nucleic acid to be detected can be identified or be distinguished from other components of a mixture (for example, cell extracts, genomic DNA, etc.). Said second region of the oligonucleotide is defined in the first aspect of the invention.

In preferred embodiments, specific hybridization is carried out under highly restrictive conditions or under moderately restrictive conditions. Suitable conditions for said hybridization to occur are illustratively shown in Examples 8, 9, 10, 11 and 12 of the application. For example, hybridization between the target nucleic acid molecule and the second region of the oligonucleotide forming the nanoparticle of the invention takes place when about 50 μl of gold nanoparticles at an approximate concentration of 10 nM are contacted in about 100 μl of water or any suitable saline

buffer with about 15 μl of the sample containing the nucleic acid of interest at a concentration of between 1 to 200 ng/μl.

In a preferred embodiment, the second nucleotide region of the oligonucleotide forming part of the nanoparticle is 100% complementary to the corresponding region of the nucleic acid to be detected. In another preferred embodiment, the second nucleotide region of the oligonucleotide forming part of the nanoparticle is at least 50%, at least 60%, at least 70%, at least 80%, at least 90%, at least 95%, at least 99% complementary to the corresponding region of the nucleic acid to be detected.

As a result of said hybridization between the second region of the oligonucleotide forming the nanoparticle of the invention and a target sequence, a conformational change occurs in said oligonucleotide because the hybrid that is formed between said second region of the oligonucleotide and the target molecule is thermodynamically more stable than the nanoparticle, wherein said second region of the oligonucleotide does not hybridize with said target sequence. Thermodynamic stability can be determined by means of parameters known by the person skilled in the art, such as Gibbs free energy. Said conformational change refers to the fact that the oligonucleotide forming the nanoparticle of the invention switches from "conformation I" to "conformation II". As it is used herein, the term "conformation I of the oligonucleotide forming the nanoparticle of the invention" refers to a conformation wherein the oligonucleotide has a hairpin-type structure due to hybridization between the first and third regions of said oligonucleotide. In this conformation, the hydrophobic molecule bound to one end of the oligonucleotide is not exposed to the solvent. Said conformation I is the native conformation of the oligonucleotide forming the nanoparticle of the invention. As it is used herein, the term "conformation II of the oligonucleotide forming the nanoparticle of the invention" refers to the conformation adopted by said oligonucleotide when the second region of the oligonucleotide forming said nanoparticle hybridizes with a target sequence. As a result of said hybridization, the Watson-Crick bonds formed between the complementary nucleotides of the first and third regions of said oligonucleotide break. In this conformation II, the hydrophobic group bound to the oligonucleotide forming the nanoparticle of the invention is exposed to the solvent. The term "exposed to the solvent" refers to the fact that said group contacts with the solvent.

In a particular embodiment, said first step of the method for detecting a nucleic acid in a sample comprises contacting metal nanoparticles of the invention with a sample containing the nucleic acid to be detected, wherein the metal forming said nanoparticles is selected from of the group consisting of gold, silver, copper, aluminum, platinum, iron, cobalt, palladium and combinations thereof. In a more particular embodiment, the method for detecting a nucleic acid in a sample comprises contacting gold nanoparticles with said sample containing the nucleic acid to be detected.

As described in the context of the first and second aspects of the invention, the oligonucleotide forming the nanoparticle of the invention is modified with a hydrophobic molecule at one end and with a suitable group for the binding of the oligonucleotide to said metal nanoparticle at the end opposite to where the hydrophobic molecule is located. In a particular embodiment, said suitable group for the binding of the oligonucleotide to said nanoparticle is selected from a functional group containing a sulfur atom and a polyadenine sequence.

In a particular embodiment, said nanoparticle is modified with an oligonucleotide containing a functional group containing a sulfur atom at the end opposite the end where it is modified with said hydrophobic molecule. In a particular and preferred embodiment of the invention, the group containing the sulfur atom is bound to the 3' end of the oligonucleotide and the hydrophobic molecule is bound to the 5' end of the oligonucleotide. In another particular embodiment, the group containing the sulfur atom is bound to the 5' end of the oligonucleotide and the hydrophobic molecule is bound to the 3' end of the oligonucleotide.

In a particular embodiment, said group containing a sulfur atom bound to the oligonucleotide forming the nanoparticle of the invention is selected from a thiol group, a dithiolane group and an isocyanide group. In an even more particular embodiment, said group containing sulfur is a dithiolane group, and preferably said group is 1,2-dithiolane.

In a preferred embodiment, the group containing the sulfur atom and the end of the oligonucleotide are bound through a spacer sequence. In an even more preferred embodiment, the spacer sequence is a polyadenine sequence.

In another particular embodiment, said nanoparticle is modified with an oligonucleotide containing a polyadenine sequence at the end opposite the end where it is modified with said hydrophobic molecule. In a particular embodiment of the invention, the polyadenine sequence is bound to the 3' end of the oligonucleotide and the hydrophobic molecule is bound to the 5' end of the oligonucleotide. In another particular embodiment, the polyadenine sequence is bound to the 5' end of the oligonucleotide and the hydrophobic molecule is bound to the 3' end of the oligonucleotide.

In another particular embodiment, said hydrophobic molecule bound to the oligonucleotide forming the nanoparticle of the invention is a lipid, particularly a steroid lipid, and even more particularly a cholesterol molecule.

In another particular embodiment, the first step of the method for detecting a nucleic acid in a sample comprises contacting a nanoparticle according to the present invention with said sample. The second step of the method defined in the third aspect of the invention comprises determining the surface plasmon band intensity of the nanoparticles. The term "surface plasmon", "surface plasmon band" or "surface plasmon resonance" refers to the collective vibration of the electrons of the metal surface, producing an absorption band in the ultraviolet-visible spectrum (typical of the metal and of the size of the nanoparticles) at the wavelength where the resonance condition occurs in said electrons. The surface plasmon band is very sensitive to changes on the surface of the metal, particularly to changes in the dielectric function

of the medium. The surface plasmon of the nanoparticles of the invention is determined by (i) the size of said nanoparticles and (ii) by the conformation adopted by the oligonucleotide forming part of the nanoparticle, wherein the hydrophobic molecule is close to the metal surface of said nanoparticle when the nanoparticle is in conformation I, and wherein the hydrophobic molecule is far from the metal surface when the nanoparticle is in conformation II. As discussed above, as a result of hybridization between the second region of the oligonucleotide and a target sequence, a conformational change occurs in said oligonucleotide forming the nanoparticle of the invention and said nanoparticle switches to a conformation wherein the hydrophobic group bound to the oligonucleotide forming the nanoparticle of the invention is exposed to the solvent. In said conformation, the nanoparticles tend to associate with one another (aggregate) due to the hydrophobic interactions between them, which will be generated to minimize interaction between the hydrophobic molecule and the aqueous solution in which the present method is carried out. As it is used herein, the term "aggregation" refers to the process whereby two or more individual nanoparticles aggregate as a result of the interaction between them. As a result of said aggregation, aggregates formed by dimers, trimers, and aggregates of a higher order will be generated.

The determination of the surface plasmon band intensity must be carried out under suitable conditions that allow correct hybridization between the second region of the oligonucleotide forming the nanoparticle of the invention with the target sequence and nanoparticle aggregate formation. The conditions under which the method of the third aspect of the present invention is carried out will preferably be those reaction conditions in which spontaneous nanoparticle aggregation does not occur, i.e., those conditions in which in the absence of the nucleic acid target molecule, the nanoparticles are in suspension and individualized. As the person skilled in the art will understand, said conditions will depend on the temperature, the pH of the medium, nanoparticle concentration, composition of the reaction medium or on the hybridization time of the molecules.

The surface plasmon band intensity of the nanoparticles of the invention can be determined by any conventional direct or indirect technique known in the state of the art. For example, the surface plasmon can be determined by means of any spectroscopic technique known in the state of the art, such as surface plasmon resonance (SPR) spectroscopy, whereby the metal atoms are subjected to an electromagnetic beam, or surface plasmon resonance fluorescence spectroscopy (SPFS) based on the detection of the variation of the refractive index of the metal atoms when they are subjected to a photon beam.

The authors of the present invention have observed that the change in surface plasmon band intensity of the nanoparticles of the invention in the presence of a target sequence is due to a change in conformation of said nanoparticles and to the formation of aggregates of said nanoparticles

In a particular embodiment, the surface plasmon band intensity is determined by means of spectroscopy. Examples of spectroscopy techniques for indirectly determining the surface plasmon band intensity that can be used in the present invention include, but are not limited to, Raman spectroscopy, UV-visible spectroscopy, infrared spectroscopy, etc. Spectroscopy is based on detecting the absorption or emission of electromagnetic radiation at certain wavelengths that is related to the energy level involved in a quantum transition.

Raman and infrared (IR) techniques measure in the same region of the electromagnetic radiation spectrum that of the vibrational energy of the molecules, although both techniques detect different phenomena occurring with the chemical bonds. The difference is that the IR technique provides the signal of the changes in polarity of the excited bond according to one vibration or another, and the Raman technique provides the change in polarizability experienced by the bond.

In a preferred embodiment, the surface plasmon band intensity of the nanoparticles of the invention is detected by means of UV-Visible spectroscopy. Said technique is based on determining the surface plasmon resonance in a suitable wavelength depending on the metal forming the nanoparticles. Thus, in the event that the nanoparticles are gold nanoparticles, the determination of surface plasmon resonance is carried out at a wavelength of about 530 nm. If desired, absorption can be determined in a certain wavelength range, for example between 400 and 650 nm, wherein there will be a wavelength at which absorption is maximum. Maximum radiation absorbed by the nanoparticles of the invention, particularly gold nanoparticles, is preferably determined at a wavelength of 530 nm. The absorbance measurement can be carried out after a time interval that is considered suitable. For example, absorbance can be determined after a period of time that is optimal for hybridization to occur between the second region of the oligonucleotide forming the nanoparticle and the target nucleic acid to be determined in the sample and for nanoparticle aggregates to be formed. In a preferred embodiment, the absorbance measurement is determined after a sample-nanoparticle incubation period of about 4 hours. In another preferred embodiment, the absorbance measurement is determined after a sample-nanoparticle incubation period of about 16 hours.

In another preferred embodiment, the surface plasmon band intensity is determined by the observation of a change in color saturation of the sample containing said nanoparticles given that the color conferred to the sample is due to the physical and chemical properties of the nanoparticle fraction in suspension. Therefore, a decrease in color saturation of the sample will be indicative of nanoparticle aggregate formation and, as a result, of a decrease in the surface plasmon band.

According to the present method of the invention for detecting the presence of a nucleic acid in a sample, the surface plasmon band intensity of the nanoparticles of the invention is determined in a sample containing said nucleic acid and in a sample not containing said nucleic acid, wherein a decrease in the surface plasmon band intensity in the sample containing the nucleic acid to be detected with respect to the surface plasmon band intensity determined in the sample not containing said nucleic acid to be detected is indicative of the presence of said nucleic acid in said sample. The surface plasmon band intensity in said sample not containing the nucleic acid to be detected can be determined by any suitable technique. The determination of the surface plasmon band intensity in the sample not containing the target nucleic acid will preferably be carried out by means of the same technique used for detecting the surface plasmon band intensity in the sample containing said nucleic acid. In a preferred embodiment of the invention, the determination of the degree of aggregation of a nanoparticle according to the present invention in a sample containing the nucleic acid to be detected is carried out at the same time as the determination of said surface plasmon band intensity of the nanoparticles of the invention in a sample not containing the nucleic acid to be detected. Alternatively, the invention contemplates the surface plasmon band intensity of both samples being determined at different times spaced out by a suitable time interval; for example, 1 hour, 2 hours, 5 hours, 10 hours, 12 hours, 24 hours, two days, three days, five days, ten days, fifteen days, a month, six months, a year or more can lapse between the determination of the surface plasmon band intensity of a nanoparticle according to the invention in a sample containing the sample of nucleic acid to be detected and the determination of the surface plasmon band intensity of a nanoparticle according to the invention in a sample not containing said nucleic acid to be detected. If the determination of the surface plasmon band intensity in said samples is carried out at times spaced out by a time interval, then it is advisable to store the nanoparticles under suitable conditions to avoid their degradation.

As it is used herein, the term "a decrease in the surface plasmon band intensity" refers to an at least 0.01-fold, 0.05-fold, 0.075-fold, 0.1-fold, 0.5-fold, 1-fold, 1.5-fold, 2-fold, 5-fold, 10-fold, 15-fold, 20-fold, 30-fold, 40-fold, 50-fold, 60-fold, 70-fold, 80-fold, 90-fold, or 100-fold decrease in said surface plasmon band intensity of the nanoparticles of the invention determined in the sample containing said nucleic acid with respect to said surface plasmon band intensity of the nanoparticles determined in the sample not containing said nucleic acid.

In a preferred embodiment of the invention, the surface plasmon band intensity of the nanoparticles is determined by means of UV-Visible spectroscopy in a suitable range (for example between 450-600 nm). After the time that is suitable for hybridization to occur between the second region of the oligonucleotide forming the nanoparticle of the invention and the nucleic acid target molecule and for nanoparticle aggregates to form has lapsed, the absorption spectrum and absorbance at the wavelength where absorption is maximum, for example at 530 nm, are determined. The absorption spectrum and absorbance at the wavelength where absorption is maximum in a sample not containing said nucleic acid target molecule are determined under these same conditions. As a result of hybridization between the oligonucleotide forming the nanoparticle of the invention and said nucleic acid of interest there is a drop in maximum absorbance in the presence of the nucleic acid target molecule. Said drop in maximum absorbance is of about one unit with respect to said absorbance determined in said sample not containing the nucleic acid of interest. In other preferred embodiments of the invention, as a result of hybridization between the oligonucleotide forming the nanoparticle of the invention and said target nucleic acid in which said drop at the wavelength where absorbance is maximum, for example 530 nm, said drop is of at least 0.1 unit, at least 0.2 units, at least 0.3 units, at least 0.4 units, at least 0.5 units, at least 0.6 units, at least 90.7 units, at least 0.8 units, at least 0.9 units, at least 1 unit, at least 1.5 units, at least 2 units, at least 3 units, at least 4 units, at least 5 units, at least 10 units or more with respect to said absorbance determined in said sample not containing the nucleic acid of interest. The detection of a nucleic acid of interest according to the present method is illustratively shown herein in Examples 8, 9, 10, 11 and 12.

As it is used herein, the term "sample" refers to any material containing nucleic acid, for example DNA or RNA which is obtained from a biological sample or artificially synthesized. In a particular embodiment, said nucleic acid forms part of a biological sample. As it is used herein, "biological sample" refers to a tissue-, cellular- or biological fluid-type material. If the material in which the presence of a certain nucleic acid is to be determined according to the present method is a tissue or a cell, prior extraction of the nucleic acid from the sample is preferably performed using any technique suitable for that purpose. The biological sample can be treated to physically or mechanically break down the tissue or cell structure, releasing the intracellular components into an aqueous or organic solution to prepare the DNA or RNA. In a particular embodiment of the invention, said biological sample is a cell lysate. In another particular embodiment, said biological sample is a cell lysate. In another particular embodiment, said biological sample is a biological fluid.

In a particular embodiment of the invention, the nucleic acid to be determined is mRNA. Messenger RNA or "mRNA" is the ribonucleic acid containing genetic information originating from DNA to be used in the protein synthesis, i.e., it determines the order in which the amino acids will be bound.

RNA extraction can be performed by any of the methods known by the person skilled in the art, including, without being limited to, Trizol®, guanidinium salts, phenol, chloroform, etc. Said methods can be found, for example, in Sambrook et al., 2001. "Molecular cloning: a Laboratory Manual", 3rd ed., Cold Spring Harbor Laboratory Press, N.Y., Vol. 1-3. There are also commercial kits that allow extracting RNA from a sample, such as the Qiagen® RNA extraction

kit, for example. As the person skilled in the art knows, maximum precautions must be taken when working with RNA to avoid contaminations with RNases and RNA degradation.

After obtaining the RNA, an mRNA reverse transcription (RT) reaction followed by amplification by polymerase chain reaction (PCR) [RT-PCR] can be carried out if desired in order to obtain the double helix cDNA corresponding to the RNA present in the sample. As it is used herein, the term "cDNA" or "complementary DNA" refers to the single-stranded DNA that is synthesized from a single strand of RNA. As the person skilled in the art can understand, there are several cDNA synthesis methods, the most common being the use of the enzyme reverse transcriptase. Methods for carrying out cDNA synthesis are described in Sambrook *et al.,* 2001, mentioned above.

In a preferred embodiment of the invention, the present method aims to detect the presence of a gene selected from the group consisting of the GNAQ gene, the PDE6B gene and the KRAS gene.

Method for detecting the presence of a target sequence in a nucleic acid

The inventors have shown that the use of a mixture of nanoparticles of the invention in which the oligonucleotides forming part of the nanoparticles are distinguished in a single position in the loop region allows detecting the presence of nucleic acids in a sample containing a nucleotide that is complementary to the differential position between both types of nanoparticles. Detection is based on the change in emission of the plasmon of the nanoparticle when the region with the hairpin structure unfolds as a result of hybridization between the loop region thereof and the nucleic acid in the sample compared with the change experienced in the nanoparticles in which the loop region shows at least one mismatch position with the nucleic acid that is in the sample

Thus in a fourth aspect, the invention relates to a method for detecting the presence of a target sequence in a nucleic acid which comprises:

(i) separately contacting said nucleic acid with a first type of nanoparticles according to the invention and with a second type of nanoparticles according to the invention, wherein the oligonucleotide of said first type of nanoparticles contains a second region that is at least partially complementary with said target sequence, and wherein the oligonucleotide of said second type of nanoparticles is capable of hybridizing with the target sequence with less affinity than the second region of the oligonucleotide of the first type of nanoparticles, and wherein said contacting is carried out under conditions that allow said hybridization of said second regions of the oligonucleotides of the first type and of the second type of nanoparticles with the nucleic acid; and

(ii) determining the surface plasmon band intensity of said first and second types of nanoparticles in the presence of the nucleic acid

wherein a surface plasmon band intensity of the first type of nanoparticles less than the surface plasmon band intensity of the second type of nanoparticles defined in step (i) is indicative of the presence of said target sequence in the nucleic acid.

The first step of the method for detecting the presence of a target sequence in a nucleic acid comprises contacting said sample with a first type of metal nanoparticles according to the invention, wherein the oligonucleotide forming said first type of nanoparticles is at least partially complementary with said target sequence. As it is used herein, the term "target sequence" refers to a sequence of a nucleic acid formed by at least one nucleotide. In a preferred embodiment of the invention, the target sequence to be detected is a nucleotide. In another preferred embodiment, the target sequence to be detected is formed by 2, 3, 4, 5, 10, 15, 20, 30, 40, 50 or more nucleotides.

The first step of said method comprises contacting said sample with a second type of metal nanoparticles according to the invention, wherein the oligonucleotide of said second type of nanoparticles is capable of hybridizing with the target sequence with less affinity than the second region of the oligonucleotide of the first type of nanoparticles. Contacting the sample with the first type of nanoparticles according to the invention is done separately with respect to contacting said sample with the second type of nanoparticles. In preferred embodiments of the invention, the first step comprises contacting an aliquot of the sample containing the target sequence to be detected with said first type of nanoparticles, and separately contacting another aliquot of the sample with the second type of nanoparticles, wherein said aliquot is different from the aliquot that is contacted with the first type of particles.

The second region of the oligonucleotide forming the first type of nanoparticles is at least partially complementary with the target sequence to be detected, i.e., it is capable of at least partially hybridizing with the target sequence to be detected. The term "partially complementary" is defined in the third aspect of the invention. In a preferred embodiment, complementarity between the second nucleotide region of said oligonucleotide and the target sequence to be detected is 100%. In another preferred embodiment, the second nucleotide region of the oligonucleotide forming part of the first type of nanoparticles as they are defined in this inventive aspect is at least 50%, at least 60%, at least 70%, at least 80%, at least 90%, at least 95%, at least 99% complementary to the target sequence to be detected.

The second type of nanoparticles used in the method for detecting a target sequence according to the present

invention comprises a second region at least partially complementary with the target sequence to be detected. However, said second type of nanoparticles hybridizes with the target sequence with less affinity than the first type of nanoparticles. As it is used herein, the term "affinity" refers to the tendency of the nucleotides of the second region of the oligonucleotide to form the nanoparticle of the invention, to hybridize with the target sequence. The chemical affinity between two molecules can be determined by means of determining any parameter suitable for that purpose, such as Gibbs free energy or Gibbs free energy standard.

The lower affinity between the second region of the oligonucleotide forming the second type of nanoparticles and the target sequence to be detected is related to the degree of complementarity between said second region of the oligonucleotide of the nanoparticle and the target sequence to be detected. The degree of complementarity between the second region of the oligonucleotide forming the second type of nanoparticles and the target sequence is less than the complementarity existing between the second region of the oligonucleotide forming the first type of nanoparticles defined above and said target sequence.

The oligonucleotide sequences corresponding to the second region of the first and second types of nanoparticles hybridizing with the target sequence are substantially identical, i.e., they have a high degree of identity. The terms "identical" or "identity" in the context of two or more nucleic acids refers to two or more sequences that are exactly the same or that have a specified percentage of nucleotides that are exactly the same. The degree of identity between two sequences is determined using computer-implemented algorithms and methods that are widely known by the persons skilled in the art, for example, using the BLASTP algorithm (BLAST Manual, Altschul, S. *et al.,* NCBI NLM NIH Bethesda, Md. 20894, Altschul, S., et al., J., 1990, Mol. Biol. 215:403-410).

Said first step is carried out under conditions that allow hybridization between said second region of the oligonucleotide forming the first and second types of nanoparticles of the invention and the nucleic acid target sequence. The term hybridization is defined in the third aspect of the invention. Suitable conditions for said hybridization to occur are illustratively shown in Examples 8, 9, 10, 11 and 12 of the application. For example, hybridization between the target sequence and the second region of the oligonucleotide forming the first and second types of nanoparticles according to the present method takes place when about 50 μl of the first and/or second type of gold nanoparticles at a concentration of 2.7 x $10^8$ $M^{-1}cm^{-1}$ are contacted with about 15 μl of the sample containing the nucleic acid of interest at a concentration of between 1 to 200 ng/μl of a sample containing the target sequence of interest.

As mentioned in the context of the method for detecting a target nucleic acid in a sample defined in the third aspect of the present invention, hybridization of the second region of the oligonucleotide forming the first type of nanoparticles of the invention with said region of the target nucleic acid comprising the target sequence causes a conformational change in the oligonucleotide forming said first type of nanoparticles of the invention, such that in the absence of said target sequence, the oligonucleotide is in a certain conformation or "conformation I of the oligonucleotide forming the nanoparticle of the invention", which gives rise to a hairpin structure as a result of hybridization between the first and third regions of said oligonucleotide. However, in the presence of said target sequence, the second region of the oligonucleotide forming part of the first type of nanoparticles of the invention hybridizes with said target sequence. As a result of said hybridization, the bonds formed between the complementary nucleotides of the first and third regions of said oligonucleotide break. In this conformation II, the hydrophobic group bound to the oligonucleotide forming the nanoparticle of the invention is exposed to the solvent and the nanoparticles tend to form aggregates. Likewise, in the presence of the target sequence to be detected, the second region of the oligonucleotide forming the second type of nanoparticles hybridizes with said target sequence. However, the degree of complementarity between the second region of the oligonucleotide forming said second type of nanoparticles and the target sequence to be detected is less than that of the second region of the oligonucleotide forming the first type of nanoparticles and said target sequence. Therefore, hybridization of the second region of the oligonucleotide forming said second type of nanoparticles by the target sequence is less than that of the second region of the oligonucleotide of the first type of nanoparticles and said target sequence. As a result of said hybridization, the bonds formed between the complementary nucleotides of the first and third regions of said oligonucleotide break. In this conformation II, the hydrophobic group bound to the oligonucleotide forming the nanoparticle of the invention is exposed to the solvent. In a particular embodiment, said first and second types of metal nanoparticles used in the first step of the method for detecting a target sequence in a nucleic acid comprise a metal which is selected from the group consisting of gold, silver, copper, aluminum, platinum, iron, cobalt, palladium and combinations thereof. In a more particular embodiment, said first and second types of nanoparticles are gold nanoparticles.

As described in the context of the first, second and third aspects of the invention, in a particular embodiment, the oligonucleotide forming part of the nanoparticle of the invention is modified with a hydrophobic molecule at one end and with a suitable group for the binding of the oligonucleotide to said metal nanoparticle at the end opposite to where the hydrophobic molecule is located. In a particular embodiment, said suitable group for the binding of the oligonucleotide to said nanoparticle is selected from a group containing a sulfur atom and a polyadenine sequence. Therefore, in a particular embodiment, the first and second types of nanoparticles defined in the present method have said features. In a particular and preferred embodiment of the invention, said suitable group for the binding of the oligonucleotide is a

group containing a sulfur atom. In still a more particular embodiment, the group containing the sulfur atom is bound to the 3' end of the oligonucleotide and the hydrophobic molecule is bound to the 5' end of the oligonucleotide. In another particular embodiment, the group containing the sulfur atom is bound to the 5' end of the oligonucleotide and the hydrophobic molecule is bound to the 3' end of the oligonucleotide.

In another particular embodiment, said suitable group for the binding of the oligonucleotide is a polyadenine sequence. Thus, the oligonucleotide forming part of the nanoparticle of the invention is modified with a polyadenine sequence at one end, and said oligonucleotide forming the nanoparticle of the invention is modified with a hydrophobic molecule at the end opposite the end where it is modified with said polyadenine sequence. Therefore, in another particular embodiment, the first and second types of nanoparticles defined in the present method have said features. In a particular and preferred embodiment of the invention, the polyadenine sequence is bound to the 3' end of the oligonucleotide and the hydrophobic molecule is bound to the 5' end of the oligonucleotide.

In a second step, the method for detecting a target sequence in a nucleic acid comprises determining the surface plasmon band intensity of said first and second types of nanoparticles. The term "surface plasmon" and methods for determining the surface plasmon band intensity are described in the third inventive aspect. In a particular embodiment, the surface plasmon band intensity is determined by means of spectroscopy.

In a preferred embodiment, the surface plasmon band intensity of the nanoparticles of the invention is detected by means of UV-Visible spectroscopy.

According to the method defined in the invention for detecting the presence of a target sequence in a nucleic acid, said presence is determined by the surface plasmon band intensity differences between:

(i) a first type of nanoparticles according to the invention, wherein the oligonucleotide forming said nanoparticle contains a second region that is at least partially complementary with said target sequence; and
(ii) a second type of nanoparticles, wherein the oligonucleotide forming said nanoparticle contains a second region that is at least partially complementary with said target sequence and is capable of hybridizing with the target sequence with less affinity than the second region of the oligonucleotide of the first type of nanoparticles

wherein a surface plasmon band intensity of the first type of nanoparticles less than the surface plasmon band intensity obtained for the second type of nanoparticles is indicative of the presence of said target sequence in the nucleic acid.

The surface plasmon band intensity of the first and second types of nanoparticles can be determined by any suitable technique. The determination of the surface plasmon band intensity of the first type of nanoparticles and the surface plasmon band in said sample using the second type of nanoparticles will preferably be carried out by means of the same technique; the surface plasmon band intensity is preferably determined by means of spectroscopy, and even more preferably by means of UV-Visible spectroscopy. In another preferred embodiment, the surface plasmon band intensity of said first and second types of nanoparticles is determined by the observation of a change in color saturation of the sample containing said nanoparticles given that the color conferred to the sample is due to the physical and chemical properties of the nanoparticle fraction in suspension. Therefore, a decrease in color saturation of the sample, i.e., a decrease in the surface plasmon band intensity, will be the result of an increase in the number of nanoparticle aggregates in the sample.

In a preferred embodiment of the invention, the surface plasmon band intensity of either nanoparticle according to the present invention is determined at the same time. Alternatively, the invention contemplates the surface plasmon band intensity of either nanoparticle being determined at different times spaced out by a suitable time interval; for example, 1 hour, 2 hours, 5 hours, 10 hours, 12 hours, 24 hours, two days, three days, five days, ten days, fifteen days, a month, six months, a year or more can lapse between the determination of the surface plasmon band intensity of a nanoparticle in which the oligonucleotide forming part of said nanoparticle comprises a nucleotide that is complementary to the nucleotide corresponding to the position to be detected in said nucleic acid and the determination of the surface plasmon band intensity of a nanoparticle in which the oligonucleotide forming part of said nanoparticle comprises a nucleotide that is not complementary to the nucleotide corresponding to the position to be detected in said nucleic acid. If the surface plasmon band intensity in said either nanoparticle according to the present invention is determined at different times, then it is advisable to store the sample under suitable conditions to avoid their degradation. Suitable conditions to avoid degradation of the sample are described in the context of the method for detecting a nucleic acid in a sample defined in the third aspect of the invention. In this case, it is advisable to store the nanoparticles under conditions that avoid deterioration thereof. The nanoparticles of the invention are stable over time and can be stored at a temperature between about -20°C and 4°C.

As it is used herein, the expression "a decrease in the surface plasmon band intensity of the nanoparticles" refers to an at least 0.01-fold, 0.05-fold, 0.075-fold, 0.1-fold, 0.5-fold, 1-fold, 1.5-fold, 2-fold, 5-fold, 10-fold, 15-fold, 20-fold, 30-fold, 40-fold, 50-fold, 60-fold, 70-fold, 80-fold, 90-fold, or 100-fold decrease in said surface plasmon band intensity of the first type of nanoparticles of the invention with respect to said plasmon band intensity of the second type of nanoparticles.

The method for detecting a target sequence in a nucleic acid according to the present invention can be used for detecting alterations in a nucleic acid sequence by determining the differences between the surface plasmon band intensity of a first type of nanoparticles in which the oligonucleotide is complementary to the unaltered sequence and the surface plasmon band intensity of a second type of nanoparticles in which the oligonucleotide is complementary to the altered sequence.

As it is used herein, "alteration" or "altered sequence" refers to a mutation, i.e., to substitutions, insertions or deletions, occurring at the nucleotide sequence level. As it is used herein, "insertion" is understood as the gaining of one or more nucleotides. Insertion includes duplications, consisting of the repetition of a DNA segment from within a sequence, being able to be produced three (triplication) or more times. As it is used herein, "deletion" is understood as the loss of one or more nucleotides. Deletions can be complete or partial deletions. As it is used herein, "complete deletion" of a gene sequence refers to the loss of 100% of the nucleotides making up the nucleotide sequence of said gene. As it is used herein, "partial deletion" of a gene sequence refers to the loss of at least 0.5%, 1%, 5%, 10%, 20%, 40%, 50%, 60%, 70%, 80%, 90% or 99% of the nucleotides making up the nucleotide sequence of said gene.

The authors of the present invention have observed that when the target sequence to be detected contains a guanine or cytosine residue, the aggregation differences seen when the sample is contacted with a first type of nanoparticles of the invention in which the second region of the oligonucleotide contains a nucleotide complementary to said guanine or cytosine (cytosine or guanine, respectively) and when the sample is contacted with a second type of nanoparticles of the invention in which the oligonucleotide contains a nucleotide that is not complementary to said guanine or cytosine and hybridize with the target sequence with less affinity, are greater than those seen when the target sequence to be detected contains a residue that is not guanine or cytosine (for example, adenine or thymine).

In a particular embodiment, the method for detecting a target sequence in a nucleic acid aims to detect a certain nucleotide in said nucleic acid, preferably cytosine or guanine.

As discussed above, the sequences of the oligonucleotides of the first and second types of nanoparticles are substantially identical. In a particular embodiment of the invention, the sequence of the second region of the oligonucleotide of the second type of nanoparticles is a variant of the second region of the oligonucleotide of the first type of nanoparticles, i.e., the sequences of the oligonucleotides of the first and second types of nanoparticles of the invention differ by a single position in the second region of said oligonucleotides. In a more particular embodiment, said single position is guanine or cytosine in the oligonucleotide of the first type of nanoparticles. In another particular embodiment, the sequences of the oligonucleotides of the first and second types of nanoparticles of the invention differ by two positions, by three positions, by four positions, by five positions, by six positions, by seven positions, by eight positions, by nine positions, by ten positions or more, in the second region of said oligonucleotides.

Thus in a preferred embodiment, the nucleotide to be determined is guanine or cytosine.

In a preferred embodiment of the invention, the surface plasmon band intensity of the first and second types of nanoparticles is determined by means of UV-Visible spectroscopy in a suitable range (for example between 450-600 nm). The target sequence to be detected is preferably guanine or cytosine. In a preferred embodiment, the oligonucleotide of the first type of nanoparticles comprises guanine or cytosine complementary to the target sequence, and the oligonucleotide of the second type of nanoparticles has a sequence identical to the sequence of the oligonucleotide of the first type of nanoparticles with the exception of said guanine or cytosine. Once the suitable time for hybridization to take place between the second region of the oligonucleotide forming the first type of nanoparticles and the target sequence to be detected and nanoparticle aggregate formation has lapsed, the absorption spectrum and absorbance at the wavelength where absorption is maximum, for example at 530 nm, are determined. The surface plasmon band intensity of the second type of nanoparticles according to the invention, in which the oligonucleotide has a sequence identical to the sequence of the oligonucleotide of the first type of nanoparticles with the exception of a nucleotide corresponding with the sequence complementary to the target sequence to be detected, is determined under these same conditions. As a result of hybridization between the second region of the first type of nanoparticles and the target sequence, a drop in absorbance with respect to the absorbance determined in said sample containing the second type of nanoparticles is seen given that said second type of nanoparticles hybridize with less affinity with the target sequence to be detected in said nucleic acid. Said drop is of about one unit with respect to said absorbance determined in said sample containing the second type of nanoparticles. In other preferred embodiments of the invention, as a result of hybridization between the oligonucleotide forming the first type of nanoparticles of the invention and said target nucleic acid in which said drop at the wavelength where absorbance is maximum, for example 530 mn, is of at least 0.1 unit, at least 0.2 units, at least 0.3 units, at least 0.4 units, at least 0.5 units, at least 0.6 units, at least 90.7 units, at least 0.8 units, at least 0.9 units, at least 1 unit, at least 1.5 units, at least 2 units, at least 3 units, at least 4 units, at least 5 units, at least 10 units or more with respect to said absorbance determined in said sample containing the second type of nanoparticles according to the invention.

In a particular embodiment, the nucleic acid containing the target sequence to be detected forms part of a biological sample. In another more particular embodiment, said biological sample is selected from the group formed by a biological fluid, a cell lysate and a tissue lysate.

In a particular embodiment of the invention, the type of nucleic acid to be determined is mRNA. Methods for obtaining RNA from a sample are described in the context of the third aspect of the invention.

In preferred embodiments, the position to be determined in a nucleic acid is in a gene or in an mRNA selected from the group consisting of GNAQ, PDE6B, KRAS, bHER2, PI3K (PIK3CA), KRAS, EGFR, c-MET, MEK, PTEN, NRAS, HRAS, FGFR1, JAK2, ABL, BRAF, ALK, NFKBETA, p53, p27, GNA11, BAP1, MDM2, CCND1, NRAS, KIT, NF1, CDKN2A, PTEN, AKT1, PI3K or BCL2.

In a preferred embodiment of the invention, the present method aims to detect the presence of the nucleotide located at position 626 in the GNAQ gene. In an even more preferred embodiment of the invention, the method for detecting the presence of said position in the GNAQ gene in a sample comprises determining the difference between the surface plasmon band intensity of a first type of nanoparticle covalently bound to an oligonucleotide comprising the sequence shown in SEQ ID NO: 5 and the surface plasmon band intensity obtained from a second type of nanoparticle covalently bound to an oligonucleotide comprising the sequence shown in SEQ ID NO: 6.

In another preferred embodiment of the invention, the present method aims to detect the presence of the nucleotide located at position 1678 in the PDE6B gene. In an even more preferred embodiment of the invention, the method for detecting the presence of said position in the PDE6B gene in a sample comprises detecting the surface plasmon band intensity of a first type of nanoparticle covalently bound to an oligonucleotide comprising the sequence shown in SEQ ID NO: 9 and comparing it with the surface plasmon band intensity obtained from a second type of nanoparticle covalently bound to an oligonucleotide comprising the sequence shown in SEQ ID NO: 10.

In another preferred embodiment of the invention, the present method aims to detect the presence of the nucleotides located in codons 12, 13 or 61 in the KRAS gene. In an even more preferred embodiment of the invention, the method for detecting the presence of said position in the KRAS gene in a sample comprises detecting the surface plasmon band intensity of a first type of nanoparticle covalently bound to an oligonucleotide comprising the sequence shown in SEQ ID NO: 1 and comparing it with the surface plasmon band intensity obtained from a second type of nanoparticle covalently bound to an oligonucleotide comprising the sequence shown in SEQ ID NO: 2.

In another preferred embodiment of the invention, the present method aims to detect the presence of the mutations responsible for the V600E, V600K, K601E, V599D, V599E, V599R or D594G mutations in the BRAF gene.

In another preferred embodiment of the invention, the present method aims to detect the presence of the mutations responsible for the Q61L, Q61K, Q61R, G12D, G13D, Y64N or D57N mutations in the NRAS gene.

In another preferred embodiment of the invention, the present method aims to detect the presence of the mutations responsible for the V560A, Y553H, D572E, L576P, K642E, K642Q, Y646D, D816V or N822I mutations in the KIT gene.

Non-limiting, illustrative examples of said method can be found in Examples 8, 9, 10, 11 and 12 of the present application.

<u>Uses of the nanoparticle of the invention</u>

In another aspect, the present invention relates to the use of a nanoparticle according to the invention for detecting a nucleic acid in a sample.

In another aspect, the invention relates to the use of a nanoparticle according to the invention for detecting a target sequence in a nucleic acid.

The nanoparticle of the invention and particular embodiments thereof are defined in the context of the first aspect of the invention.

<u>Kits of the invention</u>

In another aspect, the invention also contemplates kits comprising the nanoparticles according to the present invention as well as kits containing suitable reagents for preparing the nanoparticles of the invention.

Thus in one aspect, the invention relates to a first kit, hereinafter "first kit of the invention", comprising:

(i) a metal nanoparticle; and
(ii) an oligonucleotide comprising a first nucleotide region, a second nucleotide region and a third nucleotide region, in that order, wherein said first and third regions are at least partially complementary such that they can give rise to a hairpin-type structure by means of hybridization, and wherein said oligonucleotide is modified with a hydrophobic molecule at one end and with a suitable group for the binding of the oligonucleotide to said metal nanoparticle at the end opposite to where the hydrophobic molecule is located.

In the context of the present invention, "kit" is understood as a product of the different reagents for preparing the nanoparticles of the present invention, both in those cases in which there is a single type of nanoparticles and in

cases in which at least two types of nanoparticles are required (for example, when the presence of a nucleic acid with a certain mutation is to be identified), in which the different reagents are packaged together to allow for transport and storage. Nevertheless, if the kits defined in the present invention do not comprise the reagents necessary for putting the methods of the invention into practice, such reagents are commercially available and can be found as part of a kit. Suitable materials for packaging the components of the kit include, without being limited to, glass, plastic (polyethylene, polypropylene, polycarbonate and the like), bottles, vials, paper, sachets and the like. Kits can additionally contain instructions for using the different components in the kit. Said instructions can be in printed format or in an electronic device capable of storing instructions such that they can be read by a person, such as electronic storage media (magnetic discs, tapes and the like), optical means (CD-ROM, DVD, USB) and the like. The media can additionally or alternatively contain Internet addresses where said instructions are provided.

The first component of the first kit of the invention comprises a metal nanoparticle. The term "metal nanoparticle" and its particular embodiments are defined in the context of the first aspect of the invention and are incorporated by reference in this inventive aspect.

The second component of the first kit of the invention comprises an oligonucleotide comprising a first nucleotide region, a second nucleotide region and a third nucleotide region, in that order, wherein said first and third regions are at least partially complementary such that they can give rise to a hairpin-type structure by means of hybridization, and wherein said oligonucleotide is modified with a hydrophobic molecule at one end and with a suitable group for the binding of the oligonucleotide to said metal nanoparticle at the end opposite to where the hydrophobic molecule is located. In a particular embodiment, said suitable group for the binding of the oligonucleotide to said metal nanoparticle is selected from a functional group containing a sulfur atom and a polyadenine sequence.

In a particular embodiment, said oligonucleotide is modified with a functional group containing a sulfur atom at the end opposite the end where it is modified with said hydrophobic molecule. In another particular embodiment, said oligonucleotide is modified with a polyadenine sequence at the end opposite the end where it is modified with said hydrophobic molecule. The terms "oligonucleotide", "hairpin-type structure", "group containing a sulfur atom", "polyadenine sequence" and "hydrophobic molecule", as well as particular embodiments thereof, are defined above in the context of the second aspect of the invention.

In a particular embodiment, the first kit of the invention additionally comprises suitable reagents for carrying out functionalization between the oligonucleotide and the metal nanoparticle of said kit. In a particular embodiment, said first kit additionally comprises suitable reagents for forming a covalent bond between the sulfur atom of the functional group containing said sulfur atom of the oligonucleotide and the metal atom of said metal nanoparticle. In another particular embodiment, said first kit additionally comprises suitable reagents for adsorption of the adenine nucleotides of said polyadenine sequence in the metal atoms of the nanoparticle. In still a more particular embodiment, the first kit of the invention comprises suitable reagents for carrying out the method for obtaining a metal nanoparticle according to the invention. Illustrative, non-limiting examples of said reagents include buffered solutions such as phosphate buffered saline (PBS), saline solutions (sodium chloride, sodium phosphate, potassium chloride, potassium phosphate), etc.

In a preferred embodiment, the second component of the first kit of the invention comprises an oligonucleotide comprising the sequence shown in SEQ ID NO: 5, SEQ ID NO: 9, SEQ ID NO: 1.

The present invention also contemplates a kit, hereinafter "second kit of the invention", comprising a metal nanoparticle according to the present invention.

The present invention also contemplates a kit, hereinafter "third kit of the invention", comprising:

(i) a metal nanoparticle;

(ii) a first oligonucleotide comprising a first nucleotide region, a second nucleotide region and a third nucleotide region, in that order, wherein said first and third regions are at least partially complementary such that they can give rise to a hairpin-type structure by means of hybridization, and wherein said oligonucleotide is modified with a hydrophobic molecule at one end and with a suitable group for the binding of the oligonucleotide to said metal nanoparticle at the end opposite to where the hydrophobic molecule is located; and

(iii) a second oligonucleotide comprising a first nucleotide region, a second nucleotide region and a third nucleotide region, in that order, wherein the first and third regions have sequences identical to the first and third regions of the first oligonucleotide, and wherein the second region is a variant of the second region of the first oligonucleotide, and wherein said oligonucleotide is modified with said hydrophobic molecule at one end and with a suitable group for the binding of the oligonucleotide to said metal nanoparticle at the end opposite to where the hydrophobic molecule is located.

In a particular embodiment, a suitable group for the binding of the oligonucleotide to said metal nanoparticle is selected from a functional group containing a sulfur atom and a polyadenine sequence. Therefore in a particular embodiment of the invention, said first and/or second oligonucleotide is modified with a functional group containing a sulfur atom at the end opposite the end where it is modified with said hydrophobic molecule. In another particular embodiment,

said first and/or second oligonucleotide is modified with a polyadenine sequence at the end opposite the end where it is modified with said hydrophobic molecule

In a particular embodiment, the third kit of the invention additionally comprises suitable reagents for carrying out the functionalization between said first and second oligonucleotides and the metal nanoparticle. In a particular embodiment, said third kit of the invention additionally comprises suitable reagents for forming a covalent bond between the sulfur atom of the functional group containing said sulfur atom of the first oligonucleotide and the metal atom of said metal nanoparticle, giving rise to a first nanoparticle according to the present invention, and/or for forming a covalent bond between the sulfur atom of the functional group containing said sulfur atom of the second oligonucleotide and the metal atom of said metal nanoparticle, giving rise to a second nanoparticle according to the present invention. In another particular embodiment, said third kit of the invention additionally comprises suitable reagents for adsorption of the adenine nucleotides of said polyadenine sequence of the first oligonucleotide on the metal atoms of said metal nanoparticle, giving rise to a first nanoparticle according to the present invention, and/or for the adsorption of the adenine nucleotides of said polyadenine sequence of the second oligonucleotide on the metal atoms of said metal nanoparticle, giving rise to a second nanoparticle according to the present invention. In still a more particular embodiment, the third kit of the invention comprises suitable reagents for carrying out the method for obtaining a metal nanoparticle according to the invention. Illustrative, non-limiting examples of said reagents are described in the context of the first and second kits of the invention and are used with the same meaning in the context of the third kit of the invention.

In preferred embodiments, the first and second oligonucleotides of the third kit of the invention hybridize specifically with a gene selected from the group consisting of GNAQ, PDE6B, KRAS, bHER2, PI3K (PIK3CA), KRAS, EGFR, c-MET, MEK, PTEN, NRAS, HRAS, FGFR1, JAK2, ABL, BRAF, ALK, NFKBETA, p53, p27 or BCL2 in the regions thereof containing mutations of clinical interest.

In a preferred embodiment, the first and second oligonucleotides of the third kit of the invention hybridize specifically with the KRAS gene in a region thereof containing codons 12, 13 or 61. In a preferred embodiment, the first and second oligonucleotides of the third kit of the invention hybridize specifically with the GNAQ gene in a region thereof containing the nucleotide located at position 626. In a preferred embodiment, the first and second oligonucleotides of the third kit of the invention hybridize specifically with the PDE6B gene in a region thereof containing the nucleotide located at position 1678 in the PDE6B gene. In a preferred embodiment of the invention, the third kit of the invention comprises a first oligonucleotide comprising the sequence shown in SEQ ID NO: 1 and a second oligonucleotide comprising the sequence shown in SEQ ID NO: 2. In a preferred embodiment of the invention, the third kit of the invention comprises a first oligonucleotide comprising the sequence shown in SEQ ID NO: 5 and a second oligonucleotide comprising the sequence shown in SEQ ID NO: 6. In a preferred embodiment of the invention, the third kit of the invention comprises a first oligonucleotide comprising the sequence shown in SEQ ID NO: 9 and a second oligonucleotide comprising the sequence shown in SEQ ID NO: 10.

In another aspect, the invention relates to a kit, hereinafter "fourth kit of the invention", comprising:

(i) a first metal nanoparticle according to the invention; and

(ii) a second metal nanoparticle according to the invention, wherein the first and third regions of the oligonucleotide have sequences identical to the first and third regions of the oligonucleotide of the first nanoparticle, and wherein the second region of the oligonucleotide of said second nanoparticle is a variant of the second region of the oligonucleotide of the second nanoparticle.

In preferred embodiments, the first and second components of the fourth kit of the invention comprise oligonucleotides hybridizing specifically with a gene selected from the group consisting of GNAQ, PDE6B, KRAS, bHER2, PI3K (PIK3CA), KRAS, EGFR, c-MET, MEK, PTEN, NRAS, HRAS, FGFR1, JAK2, ABL, BRAF, ALK, NFKBETA, p53, p27 or BCL2 in the regions thereof containing mutations of clinical interest.

In a preferred embodiment of the invention, the first component of the fourth kit of the invention comprises a metal nanoparticle according to the invention, wherein the oligonucleotide forming said nanoparticle comprises the sequence shown in SEQ ID NO: 5, and/or the second component of said kit comprises a metal nanoparticle according to the invention, wherein the oligonucleotide forming said nanoparticle comprises the sequence shown in SEQ ID NO: 6.

In a preferred embodiment of the invention, the first component of the fourth kit of the invention comprises a metal nanoparticle according to the invention, wherein the oligonucleotide forming said nanoparticle comprises the sequence shown in SEQ ID NO: 9, and/or the second component of said kit comprises a metal nanoparticle according to the invention, wherein the oligonucleotide forming said nanoparticle comprises the sequence shown in SEQ ID NO: 10.

In a preferred embodiment of the invention, the first component of the fourth kit of the invention comprises a metal nanoparticle according to the invention, wherein the oligonucleotide forming said nanoparticle comprises the sequence shown in SEQ ID NO: 1, and/or the second component of said kit comprises a metal nanoparticle according to the invention, wherein the oligonucleotide forming said nanoparticle comprises the sequence shown in SEQ ID NO: 2.

In another aspect, the invention relates to the use of the first and second kits of the invention for detecting a

nucleic acid in a sample. In a particular embodiment, the use of the first and second kits according to the invention aims to detect a nucleic acid according to the method for detecting a nucleic acid in a sample defined in the context of the third inventive aspect of the present invention.

In a preferred embodiment, the invention relates to the use of the first and second kits of the invention for detecting in a sample a gene selected from the group consisting of GNAQ gene, PDE6B gene and KRAS gene in a sample.

In another aspect, the invention relates to the use of the third and fourth kits of the invention for detecting the presence of a target sequence in a nucleic acid. In a particular embodiment, the use of the third and fourth kits according to the invention aims to detect a nucleic acid target sequence according to the method defined in the context of the fourth inventive aspect of the present invention.

In a preferred embodiment, the invention relates to the use of the third and fourth kits of the invention for detecting the presence of the nucleotide located at position 626 in the GNAQ gene.

In another preferred embodiment, the invention relates to the use of the third and fourth kits of the invention for detecting the presence of the nucleotide located at position 1678 in the PDE6B gene.

In another preferred embodiment, the invention relates to the use of the third and fourth kits of the invention for detecting the presence of the nucleotide located in codons 12, 13 and 61 of the KRAS gene.

<u>Additional aspects of the invention include:</u>

[1] A metal nanoparticle bound to an oligonucleotide, wherein said oligonucleotide comprises a first nucleotide region, a second nucleotide region and a third nucleotide region, in that order, wherein said first and third regions are at least partially complementary such that they can give rise to a hairpin-type structure by means of hybridization, and wherein said oligonucleotide is modified with a hydrophobic molecule at the end opposite the end bound to the metal nanoparticle.

[2] A metal nanoparticle according to aspect [1], wherein the oligonucleotide is bound to the nanoparticle through the sulfur atom forming part of a group containing the sulfur atom or through a polyadenine region in the oligonucleotide.

[3] A nanoparticle according to aspect [1] or [2], wherein the group containing the sulfur atom is bound to the 3' end of the oligonucleotide, and wherein the hydrophobic molecule is bound to the 5' end of the oligonucleotide.

[4] A nanoparticle according to aspect [2] or [3], wherein the group containing the sulfur atom and the end of the oligonucleotide are bound through a spacer sequence.

[5] A nanoparticle according to aspect [4], wherein said spacer sequence is a polyadenine sequence.

[6] A nanoparticle according to any of aspects [1] to [5], wherein the sulfur-metal bond is formed by reaction between the sulfur atom of a functional group containing sulfur and a metal atom of the nanoparticle.

[7] A nanoparticle according to aspect [6], wherein said functional group containing sulfur is selected from the group consisting of a thiol group, a dithiolane group and an isocyanide group.

[8] A nanoparticle according to any of aspects [1] to [7], wherein said hydrophobic molecule is a lipid.

[9] A nanoparticle according to aspect [8], wherein said lipid is a steroid lipid.

[10] A nanoparticle according to aspect [9], wherein said steroid lipid is cholesterol or a cholesterol derivative.

[11] A nanoparticle according to any of aspects [1] to [10], wherein the metal forming said nanoparticle is selected from the group consisting of gold, silver, copper, aluminum, platinum, iron, cobalt, palladium and combinations thereof.

[12] A nanoparticle according to aspect [11], wherein said metal is gold.

[13] A process for obtaining a metal nanoparticle according to any of the preceding aspects which comprises:

(i) contacting metal nanoparticles with an oligonucleotide, wherein said oligonucleotide comprises a first nucleotide region, a second nucleotide region and a third nucleotide region, in that order, wherein said first and third regions are at least partially complementary such that they can give rise to a hairpin-type structure by means of hybridization, and wherein said oligonucleotide is modified with a hydrophobic molecule at one end and with a suitable group for the binding of the oligonucleotide to said metal nanoparticle at the end opposite to where the hydrophobic molecule is located; and

(ii) maintaining the nanoparticles under suitable conditions for the binding between said oligonucleotide and the metal of the nanoparticle.

[14] A process according to aspect [13], wherein said suitable group for the binding of the oligonucleotide to said nanoparticle is selected from a functional group containing a sulfur atom and a polyadenine sequence.

[15] A process according to aspect [14], wherein the sulfur atom forms part of a group bound to the 3' end of the

oligonucleotide, and wherein the hydrophobic molecule is associated with the 5' end of the oligonucleotide.
[16] The method according to aspect [14] or [15], wherein the group containing the sulfur atom and the end of the oligonucleotide are bound through a spacer sequence.
[17] A process according to aspect [16], wherein said spacer sequence is a polyadenine sequence.
[18] A process according to aspects [15] to [17], wherein said functional group containing sulfur is selected from a thiol group, a dithiolane group, and an isocyanide group.
[19] A process according to aspects [14] to [18], wherein said hydrophobic molecule is a lipid.
[20] A process according to aspect [19], wherein said lipid is a steroid lipid.
[21] A process according to aspect [21], wherein said steroid lipid is cholesterol or a cholesterol derivative.
[22] A process according to any of aspects [13] to [21], wherein the metal forming said nanoparticles is selected from the group consisting of gold, silver, copper, aluminum, platinum, iron, cobalt, palladium and combinations thereof.
[23] A process according to aspect [22], wherein the metal forming said nanoparticles is gold.
[24] A method for detecting the presence of a nucleic acid in a sample which comprises:

(i) contacting a nanoparticle according to any of aspects [1] to [12] with said sample, wherein the second region of the oligonucleotide forming said nanoparticle is capable of specifically hybridizing with the nucleic acid to be detected, wherein said contacting is carried out under conditions that allow hybridizing said region of the oligonucleotide of the nanoparticle with the nucleic acid of said sample; and
(ii) determining the surface plasmon band intensity of the nanoparticles

wherein a decrease in the surface plasmon band intensity of the nanoparticles of the sample with respect to the surface plasmon band intensity of the nanoparticles in the absence of the nucleic acid is indicative of the presence of said nucleic acid in said sample.
[25] A method according to aspect [24], wherein said nucleic acid forms part of a biological sample.
[26] A method according to aspect [25], wherein said biological sample is selected from the group formed by a biological fluid, a cell lysate or a tissue lysate.
[27] A method according to any of aspects [24] to [26], wherein said nucleic acid is mRNA.
[28] A method according to any of aspects [24] to [27], wherein the surface plasmon band intensity according to step (ii) is determined by means of spectrophotometry.
[29] A method for detecting the presence of a target sequence in a nucleic acid which comprises:

(i) separately contacting said nucleic acid with a first type of nanoparticles according to any of aspects [1] to [12] and with a second type of nanoparticles according to any of aspects [1] to [12], wherein the oligonucleotide of said first type of nanoparticles and of contains a second region that is at least partially complementary with said target sequence, and wherein the oligonucleotide of the second type of nanoparticles contains a second region that is capable of hybridizing with the target sequence with less affinity than the second sequence of the oligonucleotide of the first type of nanoparticles, and wherein said contacting is carried out under conditions that allow hybridizing said second regions of the oligonucleotides of the first type and of the second type of nanoparticles with the nucleic acid; and
(ii) determining the surface plasmon band intensity of said first and second types of nanoparticles in the presence of the nucleic acid

wherein a surface plasmon band intensity of the first type of nanoparticles less than the surface plasmon band intensity of the second type of nanoparticles obtained in step (ii) is indicative of the presence of said target sequence in the nucleic acid.
[30] The method according to aspect [29], wherein the oligonucleotide of the first type of nanoparticles and the oligonucleotide of the second type of nanoparticles differ by a single position in the second region of said oligonucleotides.
[31] The method according to aspect [30], wherein said single position is guanine or cytosine in the oligonucleotide of the first type of nanoparticles.
[32] The method according to any of aspects [29] to [31], wherein the nucleic acid forms part of a biological sample.
[33] The method according to aspect [32], wherein said biological sample is selected from the group formed by a biological fluid, a cell lysate and a tissue lysate.
[34] Method according to any of aspects [29] to [33], wherein the nucleic acid is mRNA.
[35] Method according to any of aspects [29] to [34], wherein the surface plasmon band intensity of the step (ii) is determined by means of spectrophotometry.
[36] Use of a nanoparticle according to aspects [1] to [12] for detecting the presence of a nucleic acid in a sample

or for detecting a target sequence in a nucleic acid.
[37] A kit comprising:

(i) a metal nanoparticle; and
(ii) an oligonucleotide comprising a first nucleotide region, a second nucleotide region and a third nucleotide region, wherein said first and third regions are at least partially complementary such that they can give rise to a hairpin-type structure by means of hybridization, and wherein said oligonucleotide is modified with a hydrophobic molecule at one end and with a suitable group for the binding of the oligonucleotide to said metal nanoparticle at the end opposite to where the hydrophobic molecule is located.

[38] A kit comprising:

(i) a metal nanoparticle;
(ii) a first oligonucleotide comprising a first nucleotide region, a second nucleotide region and a third nucleotide region, in that order, wherein said first and third regions are at least partially complementary such that they can give rise to a hairpin-type structure by means of hybridization, and wherein said oligonucleotide is modified with a hydrophobic molecule at one end and with a suitable group for the binding of the oligonucleotide to said metal nanoparticle at the end opposite to where the hydrophobic molecule is located; and
(iii) a second oligonucleotide comprising a first nucleotide region, a second nucleotide region and a third nucleotide region, in that order, wherein the first and third regions have sequences identical to the first and third regions of the first oligonucleotide, and wherein the second region is a variant of the second region of the first oligonucleotide, and wherein said oligonucleotide is modified with said hydrophobic molecule at one end and with a suitable group for the binding of the oligonucleotide to said metal nanoparticle at the end opposite to where the hydrophobic molecule is located.

[39] The kit according to aspect [36], wherein said variant of the second region of the first oligonucleotide is selected from the group of a deletion or of a substitution.
[40] A kit comprising:

(i) a first metal nanoparticle according to any of aspects [1] - [12]; and
(ii) a second metal nanoparticle according to any of aspects [1] - [12], wherein the first and third regions of the oligonucleotide have sequences identical to the first and third regions of the oligonucleotide of the first nanoparticle, and wherein the second region of the oligonucleotide of said second nanoparticle is a variant of the second region of the oligonucleotide of the first nanoparticle.

The following examples are merely illustrative and must not be considered as limiting the present invention.

EXAMPLES

General methods

The reagents used were purchased from Aldrich and were used without additional purifications. Thin layer chromatography was carried out using Silica Gel 60 F254 sheets. The chromatographic columns were prepared using Silica Gel (60 Å) as the stationary phase. The mobile phase used is specified in each case. All the NMR spectra were acquired in a 300 MHz Bruker in liquid phase using the deuterated solvent indicated for each case. The chemical shifts are indicated in parts per million (ppm), and the coupling constants are indicated in Hertz (Hz).

RNA extracts

RNA extracts from mouse cells and tissues have been used herein. The required samples were obtained by means of the following protocol:

Animal tissue

The liver tissue RNA extracts were obtained from 6-8 week-old female mice carrying the rd10 missense mutation in exon 13 of the Pde6b gene. The animals were sacrificed by means of $CO_2$ asphyxiation and cervical dislocation. The liver tissue was collected under sterile conditions and frozen in liquid nitrogen. RNA was extracted from the frozen tissues using the miRNeasy kit (Quiagen, 219004) after grinding, lysating the cells with QIAzol Lysis reagent (Quiagen, 79306),

and homogenizing the sample as indicated in the supplier's instructions. All the studies with mice were approved by the UCSF Institutional Animal Care and Use Committee.

Cell culture

Human melanoma cell lines SK-Mel-2, OMM1.3, Me1202 and C918 were obtained from Dr. Urda's laboratory at the University of California San Francisco. The cells were kept in RPMI 1640 medium supplemented with 10% FBS and grown at 37°C under 5% $CO_2$ until reaching 80-90% confluence. RNA was extracted from the cells using the miRNeasy kit (Quiagen, 219004) after lysating the cells with QIAzol Lysis reagent (Qiagen, 79306), as indicated in the supplier's instructions.

Example 1. Modified solid support synthesis

5-[(3S)-1,2-dithiolan-3-yl]-N-[(1R,2S)-2-hydroxy-1-(hydroxymethyl)propyl]pentanamide (1).

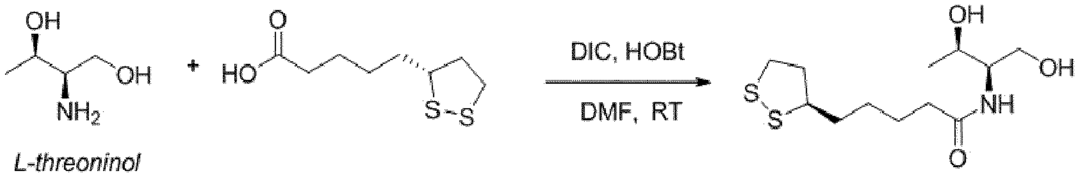


*L-threoninol*

409 mg of N-hydroxybenzotriazole (3.027 mmol, 1.1 equiv) and 468 μl of diisopropylcarbodiimide (3.026 mmol, 1.1 equiv) were added to a solution of (R)-(+)-1,2-dithiolane-3-pentanoic acid (3.301 mmol, 680 mg, 1.2 equiv) in DMF (0.5 M) at room temperature. After stirring the mixture for 10 minutes, 289 mg of L-threoninol (2.752 mmol, 1 equiv) were added and stirring continued for 16 h. The reaction was then neutralized using methanol. After removing the solvent at reduced pressure, the resulting residue was purified by means of column chromatography ($CH_2Cl_2$/MeOH 20:1) to produce 660 mg of the desired product as a pale yellow oil (82%). $^1$H-NMR (300 MHz, $CDCl_3$): δ 6.26 (d, *J*= 6.7 Hz, 1H), 4.17 (d, *J* = 6.2 Hz, 1H), 3.95 - 3.71 (m, 3H), 3.58 (dt, *J* = 13.1, 6.5 Hz, 1H), 3.48 (s, 1H), 3.26- 3.04 (m, 2H), 2.46 (td, *J* = 12.4, 6.4 Hz, 1H), 2.27 (t, *J* = 7.4 Hz, 2H), 1.91 (dq, *J* = 13.7, 6.9 Hz, 1H), 1.80 - 1.60 (m, 5H), 1.57 - 1.41 (m, 2H), 1.19 (d, *J* = 6.4 Hz, 3H). $^{13}$C-NMR (75 MHz, $CDCl_3$): δ 174.1 (C), 68.7(CH), 65.0($CH_2$), 56.4(CH), 54.7(CH), 40.3($CH_2$), 38.5($CH_2$), 36.5($CH_2$), 34.6($CH_2$), 28.8($CH_2$), 25.5($CH_2$), 20.6($CH_3$). HR-MS (ESI): *m/z* calculated for $C_{12}H_{24}NO_3S_2$ 294.1192 $[M+H]^+$, 294.1186 yielded.

N-((1R,2S)-1-{[bis(4-methoxyphenyl)(phenyl)methoxy]methyl}-2-hydroxypropyl)-5-[(3S)-1,2-dithiolan-3-yl]pentanamide (2).

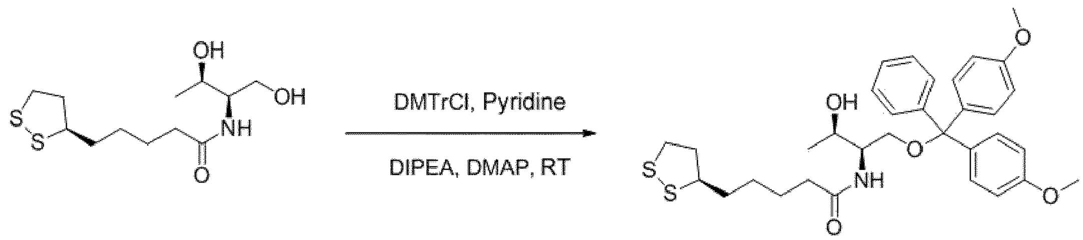


179 μl of diisopropylethylamine (1.023 mmol, 1.5 equiv), 277 mg of 4,4'-dimethoxytrityl chloride (0.819 mmol, 1.2 equiv) and a catalytic amount of dimethylaminopyridine were added to a solution of the corresponding diol (0.682 mmol, 200 mg, 1 eq.) in pyridine (0.2 M) at 0°C. After 15 min, the reaction temperature is increased to room temperature, and the mixture is stirred for another 16 h. The reaction was neutralized with methanol, and the solvent was removed at reduced pressure. The resulting residue was purified by means of column chromatography (1:2 hexane/AcOEt) to produce 270 mg of the desired product as a pale yellow foamy solid (66%). $^1$H-NMR (300 MHz, $CDCl_3$): δ 7.28 (m, 9H), 6.83 (d, *J* = 8.8 Hz, 4H), 6.03 (d, *J* = 8.7 Hz, 1H), 4.10 (m, 1H), 3.93 (dd, *J* = 8.4, 2.5 Hz, 1H), 3.79 (s, 6H), 3.62 - 3.48 (m, 1H), 3.41 (dd, *J* = 9.6, 4.3 Hz, 1H), 3.29 (dd, *J* = 9.6, 3.5 Hz, 1H), 3.23 - 3.05 (m, 3H), 2.44 (td, *J* = 12.4, 6.4 Hz, 1H),

2.23 (t, *J* = 7.3 Hz, 2H), 1.90 (td, *J* = 13.7, 6.9 Hz, 1H), 1.76 - 1.62 (m, 4H), 1.56 - 1.41 (m, 2H), 1.12 (d, *J* = 6.4 Hz, 3H). $^{13}C$-NMR (75 MHz, $CDCl_3$): δ 173.0 (C), 158.6 (2C) (C), 144.3 (C), 135.5 (C), 135.3 (C), 129.9 (2C) (CH), 129.8 (2C) (CH), 127.9 (2C) (CH), 127.8 (2C) (CH), 127.0(CH), 113.3 (4C) ($CH_3$), 86.7, 68.6(CH), 65.2($CH_2$), 60.3, 56.3(CH), 55.2(CH), 53.3(CH), 40.2($CH_2$), 38.4($CH_2$), 36.5($CH_2$), 34.6($CH_2$), 28.9(CH2), 25.5($CH_2$), 19.9($CH_3$). HR-MS (ESI) : *m/z* calculated for $C_{33}H_{41}NNaO_5S_2$ 618.2324 $[M+Na]^+$, 618.2296 yielded.

<u>Example 2: Functionalization of the solid support</u>

Step I:

10.9 mg of succinic anhydride (0.109 mmol, 1.3 eq), 20.5 μl of diisopropylethylamine (0.118 mmol, 1.4 eq) and a catalytic amount of dimethylaminopyridine (DMAP) were added to a solution of compound 2 (0.084 mmol, 50 mg, 1 eq) in anhydrous dichloromethane (0.2 M). The mixture was stirred at room temperature for 16 h, washed with water and extracted with dichloromethane. The organic phase was dried over anhydrous $MgSO_4$, filtered and concentrated at reduced pressure to produce a yellow oil.

Step II:

The product obtained in step I (0.084 mmol, 58.3 mg, 1 eq) was added to a solution of DMAP (0.126 mmol, 15.4 mg, 1.5 eq) in acetonitrile (60 mM). The mixture was stirred vigorously using a vortex stirrer and was added to a solution of 2,2'-dithiobis-(5-nitropyridine) (0.126 mmol, 39.0 mg, 1.5 eq) in anhydrous $CH_2Cl_2$ (300 μl). The mixture was stirred vigorously again and added to 33.0 mg of triphenylphosphine (0.126 mmol, 1.5 eq). The final mixture was stirred vigorously in a vortex until all the reagents were dissolved, giving rise to a red solution which was added to 500 mg of solid support (500 Å). After 2 h at room temperature, the solid support was washed with methanol (3 x 20 ml) and anhydrous acetonitrile (3 x 20 ml) and vacuum-dried. Once dry, 2 ml of a 1:1 mixture of the reagents referred to as CAP A and CAP B (CAP A: 600 μl pyridine, 500 μl anhydrous THF, 400 μl of acetic anhydride; CAP B: 1 ml anhydrous THF, 400 μl of 1-methylimidazole) were added. After 20 min at room temperature, the solid support was washed with anhydrous acetonitrile (6 x 20 ml) and vacuum-dried.

The efficiency of the preparation of the modified solid support was calculated according to the following protocol: 10 mg of the modified solid support were treated with 5 ml of a perchloric acid and ethanol solution (3:2) and stirred for 1 h. Then 500 μl of the mixture were dissolved in 2 ml of the previously prepared perchloric acid/ethanol solution, and absorbance was measured at 498 nm. Functionalization (F) efficiency was determined by means of the Lambert- Beer law:

$$F = (ABS \times V) / (\varepsilon \times g) = M / g$$

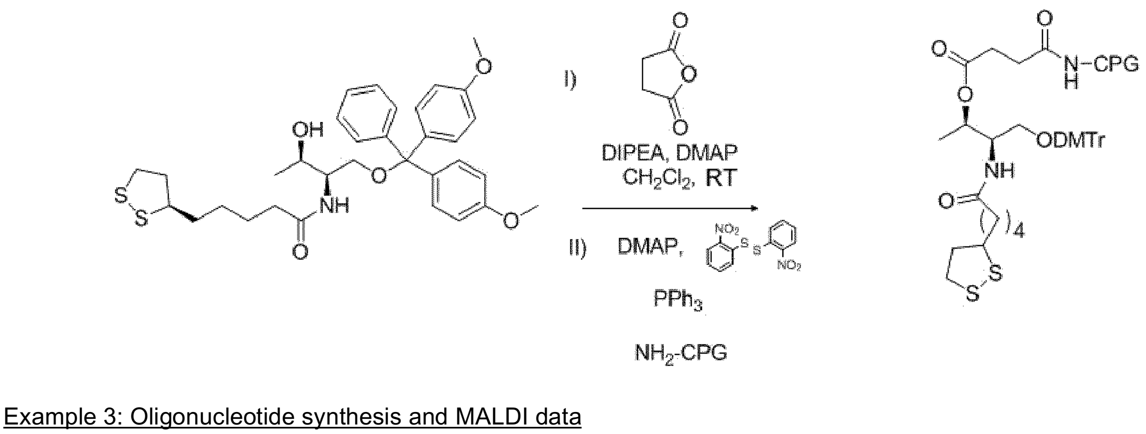


<u>Example 3: Oligonucleotide synthesis and MALDI data</u>

The oligonucleotides were prepared from commercial phosphoramidites (Link Technologies) and the modified solid support described above using a MerMade4 DNA synthesizer. The oligonucleotides were prepared at a scale of 1 μmol. After solid phase synthesis, the solid support was transferred to a glass vial and incubated at 55°C for 4 h with 2 mL of a concentrated ammonia solution (33%). After reaching room temperature, the supernatant was transferred to microcentrifuge tubes and concentrated using a centrifugal evaporator.

The oligonucleotides were purified by means of 20% acrylamide gel electrophoresis. The oligonucleotides were

extracted of the gel using an electrophoretic elution trap. Finally, they were desalted using NAP-10 columns and concentrated again in a centrifugal evaporator.

Example 4: Modification at the 5' end of the oligonucleotides

The cholesterol (Chole) functional group was introduced using the corresponding commercial phosphoramidite (Link Technologies).

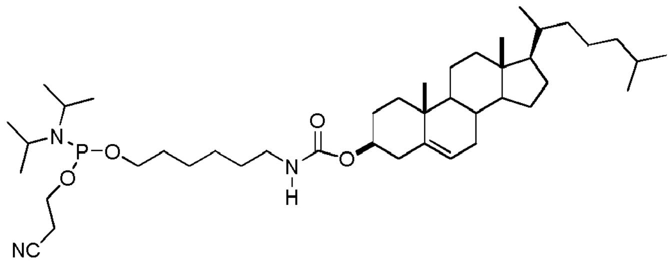

Example 5: Modification at the 3' end of the oligonucleotides

The dithiolane functional group was introduced using the solid support described above.

SEQUENCES

Table 1. Sequences according to the present invention

| SEQ ID NO: | NAME | SEQUENCE |
|---|---|---|
| 1 | KRAS-WT-Chole | 5'-Cholesterol-AGCAAGACGCCACCAGCTCCACTTGCTAAAAA-Dithiolane-3' |
| 2 | KRAS-MUT-Chole | 5'-Cholesterol-AGCAAGACGCCACAAGCTCCACTTGCTAAAAA-Dithiolane-3' |
| 3 | KRAS-WT-Target | 5'-TGGAGCTGGTGGCGT-3' |
| 4 | KRAS-MUT-Target | 5'-TGGAGCTTGTGGCGT-3' |
| 5 | GNAQ-WT-Chole | 5'-Cholesterol-CCGTCTGACCTTTGGCCCCCGACGGAAAAA-Dithiolane-3' |
| 6 | GNAQ-MUT-Chole | 5'-Cholesterol-CCGTCTGACCTTGGGCCCCCGACGGAAAAA-Dithiolane-3' |
| 7 | GNAQ-WT-Target | 5'-GGGGGCCAAAGGTCA-3' |
| 8 | GNAQ-MUT-Target | 5'-GGGGGCCCAAGGTCA-3' |
| 9 | RP-WT-Chole | 5'-Cholesterol-CTCCCCGTGGCGCCAGTTGGGAGAAAAA-Dithiolane-3' |
| 10 | RP-MUT-Chole | 5'-Cholesterol-CTCCCCGTGGCACCAGTTGGGAGAAAAA-Dithiolane-3' |
| 11 | RP-WT-Target | 5'-CAACTGGCGCCACGG-3' |
| 12 | RP-MUT-Target | 5'-CAACTGGTGCCACGG-3' |
| 13 | RP-MUT-Control | 5'-CTCCCAACTGGTGCCACGGGGAGAAAAA-Dithiolane-3' |

MALDI DATA

Table 2. MALDI data of the prepared sequences

| ENTRY | NAME | CALCULATED MASS | YIELDED MASS |
|---|---|---|---|
| 1 | KRAS-WT-Chole | 10687.3 | 10674.9 |

(continued)

| ENTRY | NAME | CALCULATED MASS | YIELDED MASS |
|---|---|---|---|
| 2 | KRAS-MUT-Chole | 10711.3 | 10702.9 |
| 3 | KRAS-WT-Target | 4680.1 | 4677.1 |
| 4 | KRAS-MUT-Target | 4655 | 4651.4 |
| 5 | GNAQ-WT-Chole | 10084.8 | 10078.3 |
| 6 | GNAQ-MUT-Chole | 10109.8 | 10116.7 |
| 7 | GNAQ-WT-Target | 4667.1 | 4655 |
| 8 | GNAQ-MUT-Target | 4643.1 | 4631.01 |
| 9 | RP-WT-Chole | 9571.42 | 9565.5 |
| 10 | RP-MUT-Chole | 9555.42 | 9534.5 |
| 11 | RP-WT-Target | 4585.0 | 4572.9 |
| 12 | RP-MUT-Target | 4569.0 | 4556.2 |
| 13 | RP-MUT-Control | 8972.01 | 8973.8 |

Example 6: Gold nanoparticle synthesis

Synthesis was carried out by means of the citrate reduction method (Ambrosi et al., 2007, Analytical chemistry, 79, 5232-40). To that end, a solution of $HAuCl_4$ (34.3 mg) in $H_2O$ (100 mL) was heated until reaching boiling temperature. A solution of sodium citrate (119.4 mg) in $H_2O$ (10 mL) was then added. The mixture was stirred for 10 min and was left to reach room temperature. The gold nanoparticles were filtered through a 4 Å filtration plate and stored at 4°C until subsequent use.

The UV/Vis spectrum of this solution showed maximum absorption centered at 520 nm. This result is consistent with the formation of gold nanoparticles that are 13 nm in diameter. This result was confirmed by means of high-resolution electron transmission microscopy, where a size distribution of 13 nm was seen.

Gold nanoparticle concentration was determined by means of the Lambert-Beer law (molar extinction coefficient at 520 nm = $2.7 \times 10^8$ $M^{-1}$ $cm^{-1}$).

Example 7: Gold nanoparticle functionalization with oligonucleotides

The gold nanoparticles were incubated with oligonucleotides SEQ ID NOs: 1, 2, 5, 6, 9, 10 and 13 for 16 h with NaCl (0.3 M). The samples were purified by means of an iterative centrifugation-supernatant removal process (3 times).

Example 8: KRAS sequence detection using oligonucleotides as the target

The gold nanoparticles (50 μL) modified with the oligonucleotides with sequence SEQ ID NO: 1 or SEQ ID NO: 2 in PBS (100 μL) were incubated at room temperature with the target sequence with sequence SEQ ID NO: 3 or SEQ ID NO: 4 for 4 h. The UV-Vis spectrum (400-650 nm) was then recorded in a quartz cuvette using 150 microliters of samples and 650 microliters of water. As shown in Figures 2 and 3, a pronounced drop in absorbance of the nanoparticles in the presence of the target KRAS sequence is seen.

Example 9: GNAQ sequence detection using oligonucleotides as the target

The gold nanoparticles (50 μL) modified with the oligonucleotides with sequence SEQ ID NO: 5 or SEQ ID NO: 6 in PBS (100 μL) containing 5 μL of 5 M NaCl were incubated at room temperature with the target sequences with sequence SEQ ID NO: 7 or SEQ ID NO: 8 for 16 h. The UV-Vis spectrum (400-650 nm) was then recorded in a quartz cuvette using 150 microliters of samples and 650 microliters of water. As shown in Figures 4 and 5, a pronounced drop in absorbance of the nanoparticles in the presence of the target GNAQ sequence is seen.

Example 10: PDE6B sequence detection using oligonucleotides as the target

The gold nanoparticles (50 μL) modified with the oligonucleotides with sequence SEQ ID NO: 9 or SEQ ID NO: 10 in a mixture of PBS (50 μL) and $H_2O$ (50 μL) were incubated at room temperature with the target sequences with sequence SEQ ID NO: 11 or SEQ ID NO: 12 for 16 h. The UV-Vis spectrum (400-650 nm) was then recorded in a quartz cuvette using 150 microliters of samples and 650 microliters of water. As shown in Figures 6 and 7, a pronounced drop in absorbance of the nanoparticles in the presence of the target PDE6B sequence is seen.

Example 11: GNAQ sequence detection in RNA extracts

Extract stock solutions with a concentration of 170 ng/μL were prepared for carrying out the detection experiments.
The gold nanoparticles (50 μL) modified with the oligonucleotides with sequence SEQ ID NO: 5 or SEQ ID NO: 6 in PBS (100 μL) and NaCl (5 M, 10 μL) were incubated for 16 h at room temperature with the RNA extracts (15 μL) obtained from 1) the C918 cell line expressing the non-mutated GNAQ gene, 2) mouse liver not containing the GNAQ gene, and 3) the OMM1.3 cell line expressing the mutated GNAQ gene. The UV-Vis spectrum was then recorded. As shown in Figures 9 and 10, a pronounced drop in absorbance of the nanoparticles in the presence of the extract containing the target sequence is seen.

Example 12: PDE6B sequence detection in RNA extracts

Extract stock solutions with a concentration of 170 ng/μL were prepared for carrying out the detection experiments.
The gold nanoparticles (50 μL) modified with the oligonucleotide with SEQ ID NO: 9 in PBS (50 μL) and $H_2O$ (50 μL) were incubated for 16 h at room temperature with the RNA extracts (20 μL) obtained from 1) mouse liver containing the mutated RP gene, 2) the SKMEL2 cell line not expressing the RP gene, and 3) the MEL202 cell line expressing the non-mutated RP gene. The UV-Vis spectrum was then recorded. As shown in Figure 11, a pronounced drop in absorbance of the nanoparticles in the presence of the extract containing the target sequence is seen.

## Claims

1. A metal nanoparticle bound to an oligonucleotide wherein said oligonucleotide comprises a first nucleotide region, a second nucleotide region and a third nucleotide region, in that order, wherein said first and third regions are at least partially complementary such that they can give rise to a hairpin-type structure by means of hybridization, and wherein said oligonucleotide is modified with a hydrophobic molecule at the end opposite the end bound to the metal nanoparticle.

2. A metal nanoparticle according to claim 1, wherein (i) the oligonucleotide contains a group containing sulfur, and wherein the binding between the nanoparticle and the oligonucleotide occurs by means of a sulfur-metal bond formed by reaction between the sulfur atom of said group and a metal atom of the nanoparticle, or (ii) wherein the oligonucleotide contains a polyadenine region, and wherein the binding between the nanoparticle and the oligonucleotide occurs by means of the adsorption of the polyadenine region on the nanoparticle.

3. A nanoparticle according to claim 2, wherein said functional group containing sulfur is selected from the group consisting of a thiol group, a dithiolane group and an isocyanide group.

4. A nanoparticle according to any of claims 2 or 3, wherein the group containing the sulfur atom is bound to the 3' end of the oligonucleotide, and wherein the hydrophobic molecule is bound to the 5' end of the oligonucleotide.

5. A nanoparticle according to any of claims 2 to 4, wherein the group containing the sulfur atom and the end of the oligonucleotide are bound through a spacer sequence.

6. A nanoparticle according to claim 5, wherein said spacer sequence is a polyadenine sequence.

7. A nanoparticle according to any of claims 1 to 6, wherein said hydrophobic molecule is a lipid.

8. A nanoparticle according to claim 7, wherein said lipid is a steroid lipid.

9. A nanoparticle according to claim 8, wherein said steroid lipid is cholesterol or a cholesterol derivative.

10. A nanoparticle according to any of claims 1 to 9, wherein the metal forming said nanoparticle is selected from the group consisting of gold, silver, copper, aluminum, platinum, iron, cobalt, palladium and combinations thereof.

11. A process for obtaining a metal nanoparticle according to any of the preceding claims which comprises:

(i) contacting metal nanoparticles with an oligonucleotide, wherein said oligonucleotide comprises a first nucleotide region, a second nucleotide region and a third nucleotide region, in that order, wherein said first and third regions are at least partially complementary such that they can give rise to a hairpin-type structure by means of hybridization, and wherein said oligonucleotide is modified with a hydrophobic molecule at one end and with a suitable group for the binding of the oligonucleotide to said metal nanoparticle at the end opposite to where the hydrophobic molecule is located; and
(ii) maintaining the nanoparticles under suitable conditions for the binding between said oligonucleotide and the metal of the nanoparticle.

12. A process according to claim 11, wherein said suitable group for the binding of the oligonucleotide to said nanoparticle is selected from a functional group containing a sulfur atom and a polyadenine sequence.

13. A method for detecting the presence of a nucleic acid in a sample which comprises:

(i) contacting the nanoparticle according to any of claims 1-10 with said sample, wherein the second region of the oligonucleotide forming said nanoparticle is capable of specifically hybridizing with the nucleic acid to be detected, wherein said contacting is carried out under conditions that allow hybridizing said region of the oligonucleotide of the nanoparticle with the nucleic acid of said sample; and
(ii) determining the surface plasmon band intensity of the nanoparticles,

wherein a decrease in the surface plasmon band intensity of the nanoparticles of the sample with respect to the surface plasmon band intensity of the nanoparticles in the absence of the nucleic acid is indicative of the presence of said nucleic acid in said sample.

14. A method for detecting the presence of a target sequence in a nucleic acid which comprises:

(i) separately contacting said nucleic acid with a first type of nanoparticles according to any of claims 1-10 and with a second type of nanoparticles according to any of claims 1-10, wherein the oligonucleotide of said first type of nanoparticles and of contains a second region that is at least partially complementary with said target sequence, and wherein the oligonucleotide of the second type of nanoparticles contains a second region that is capable of hybridizing with the target sequence with less affinity than the second sequence of the oligonucleotide of the first type of nanoparticles, and wherein said contacting is carried out under conditions that allow hybridizing said second regions of the oligonucleotides of the first type and of the second type of nanoparticles with the nucleic acid; and
(ii) determining the surface plasmon band intensity of said first and second types of nanoparticles in the presence of the nucleic acid,

wherein a surface plasmon band intensity of the first type of nanoparticles less than the surface plasmon band intensity of the second type of nanoparticles obtained in step (ii) is indicative of the presence of said target sequence

in the nucleic acid.

**15.** A kit selected from the group consisting of:

A) a kit comprising:

(i) a metal nanoparticle; and
(ii) an oligonucleotide comprising a first nucleotide region, a second nucleotide region and a third nucleotide region, wherein said first and third regions are at least partially complementary such that they can give rise to a hairpin-type structure by means of hybridization, and wherein said oligonucleotide is modified with a hydrophobic molecule at one end and with a suitable group for the binding of the oligonucleotide to said metal nanoparticle at the end opposite to where the hydrophobic molecule is located;

B) a kit comprising:

(i) a metal nanoparticle;
(ii) a first oligonucleotide comprising a first nucleotide region, a second nucleotide region and a third nucleotide region, in that order, wherein said first and third regions are at least partially complementary such that they can give rise to a hairpin-type structure by means of hybridization, and wherein said oligonucleotide is modified with a hydrophobic molecule at one end and with a suitable group for the binding of the oligonucleotide to said metal nanoparticle at the end opposite to where the hydrophobic molecule is located; and
(iii) a second oligonucleotide comprising a first nucleotide region, a second nucleotide region and a third nucleotide region, in that order, wherein the first and third regions have sequences identical to the first and third regions of the first oligonucleotide, and wherein the second region is a variant of the second region of the first oligonucleotide, and wherein said oligonucleotide is modified with said hydrophobic molecule at one end and with a suitable group for the binding of the oligonucleotide to said metal nanoparticle at the end opposite to where the hydrophobic molecule is located;
and

C) a kit comprising:

(i) a first metal nanoparticle according to any of claims 1-10; and
(ii) a second metal nanoparticle according to any of claims 1-10, wherein the first and third regions of the oligonucleotide have sequences identical to the first and third regions of the oligonucleotide of the first nanoparticle, and wherein the second region of the oligonucleotide of said second nanoparticle is a variant of the second region of the oligonucleotide of the first nanoparticle.

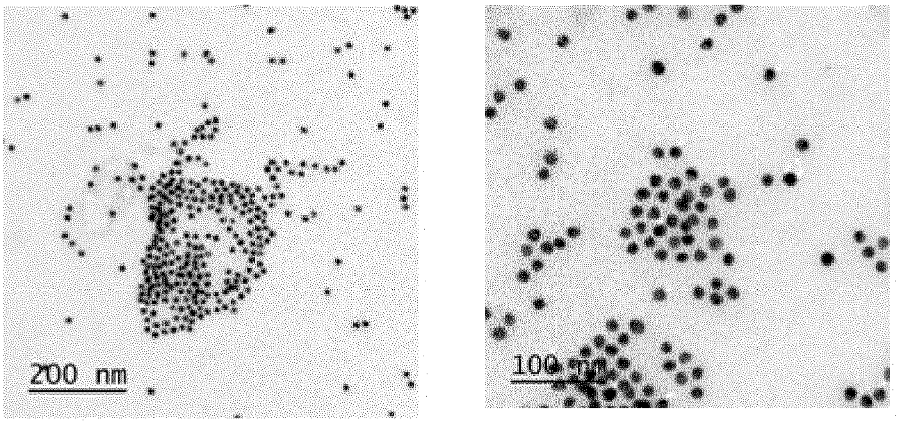
200 nm
100 nm

Fig. 1

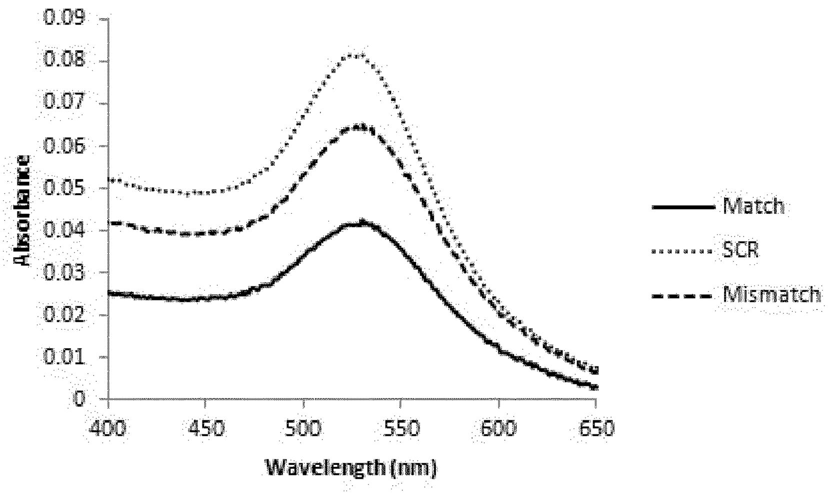
0.09
0.08
0.07
0.06
0.05
0.04
0.03
0.02
0.01
0
Absorbance
400
450
500
550
600
650
Wavelength (nm)
Match
SCR
Mismatch

Fig. 2

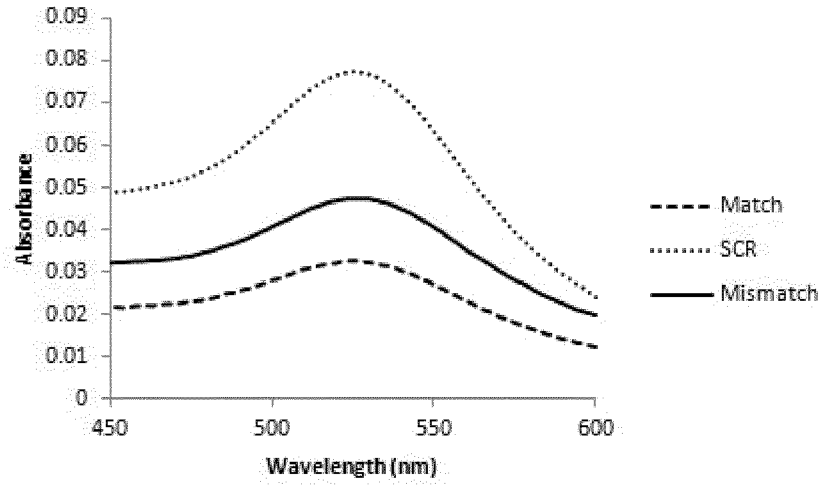
0.09
0.08
0.07
0.06
0.05
0.04
0.03
0.02
0.01
0
Absorbance
450
500
550
600
Wavelength (nm)
Match
SCR
Mismatch

Fig. 3

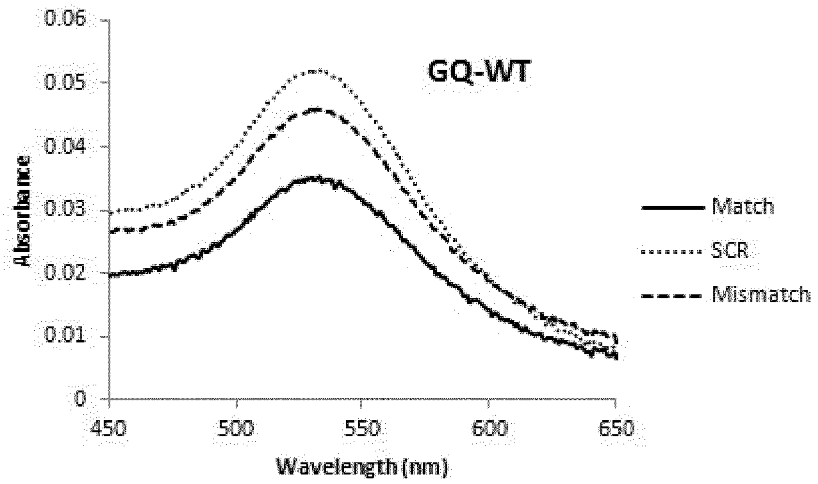
GQ-WT
Absorbance
0.06
0.05
0.04
0.03
0.02
0.01
0
450
500
550
600
650
Wavelength (nm)
Match
SCR
Mismatch

Fig. 4

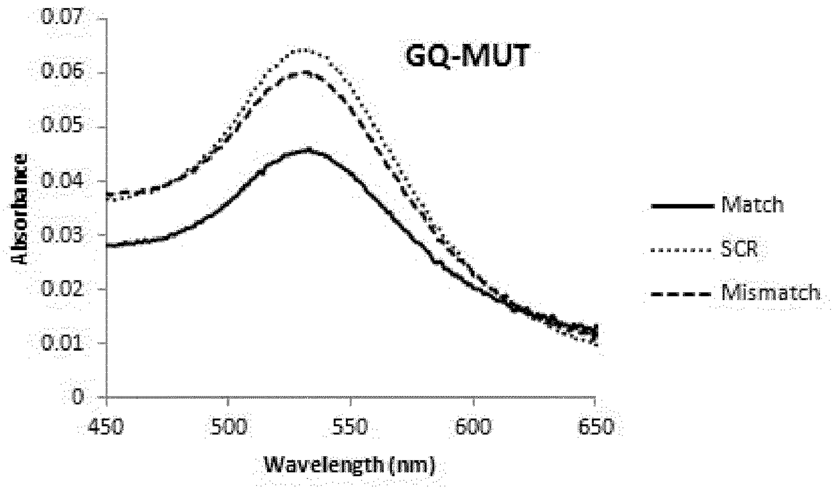
GQ-MUT
Absorbance
0.07
0.06
0.05
0.04
0.03
0.02
0.01
0
450
500
550
600
650
Wavelength (nm)
Match
SCR
Mismatch

Fig. 5

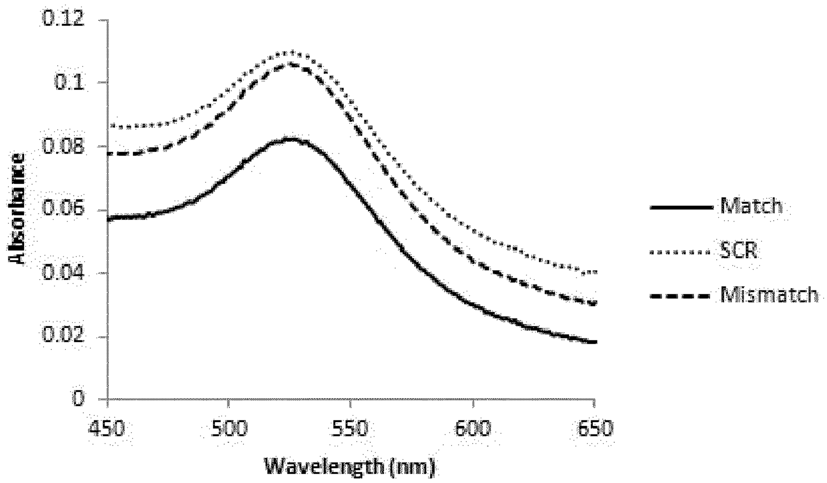
0.12
0.1
0.08
0.06
0.04
0.02
0
Absorbance
450
500
550
600
650
Wavelength (nm)
Match
SCR
Mismatch

Fig. 6

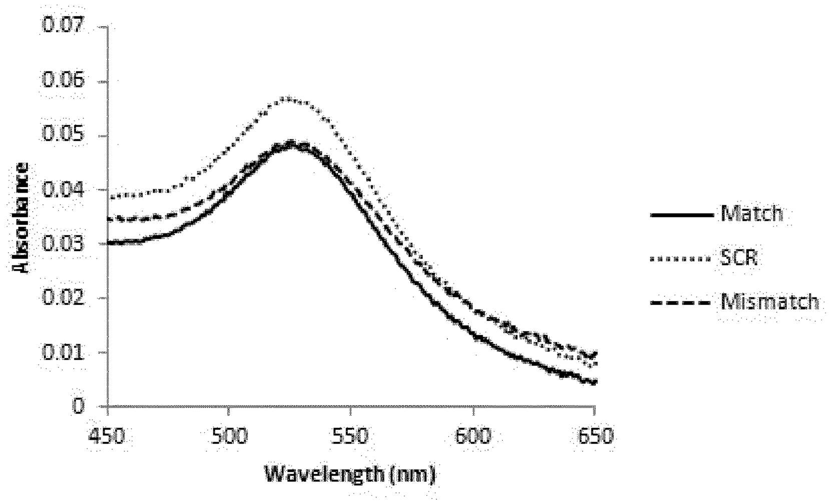
0.07
0.06
0.05
0.04
0.03
0.02
0.01
0
Absorbance
450
500
550
600
650
Wavelength (nm)
Match
SCR
Mismatch

Fig. 7

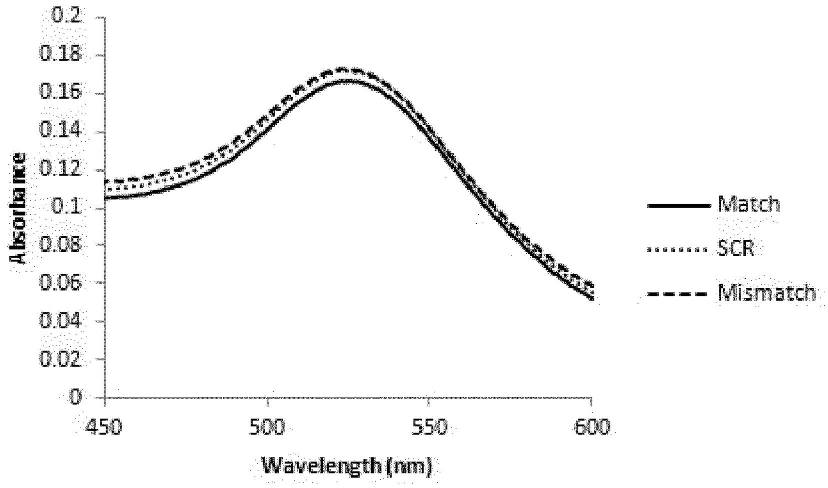
0.2
0.18
0.16
0.14
0.12
0.1
0.08
0.06
0.04
0.02
0
Absorbance
450
500
550
600
Wavelength (nm)
Match
SCR
Mismatch

Fig. 8

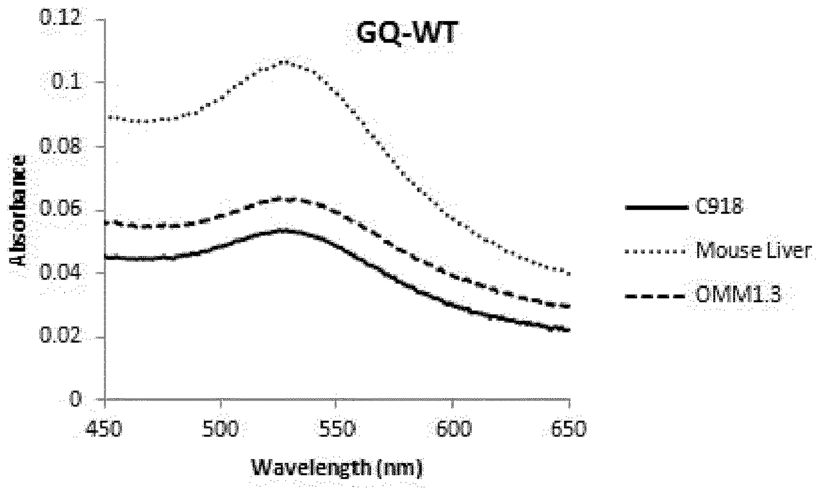
GQ-WT
0.12
0.1
0.08
0.06
0.04
0.02
0
Absorbance
450
500
550
600
650
Wavelength (nm)
C918
Mouse Liver
OMM1.3

Fig. 9

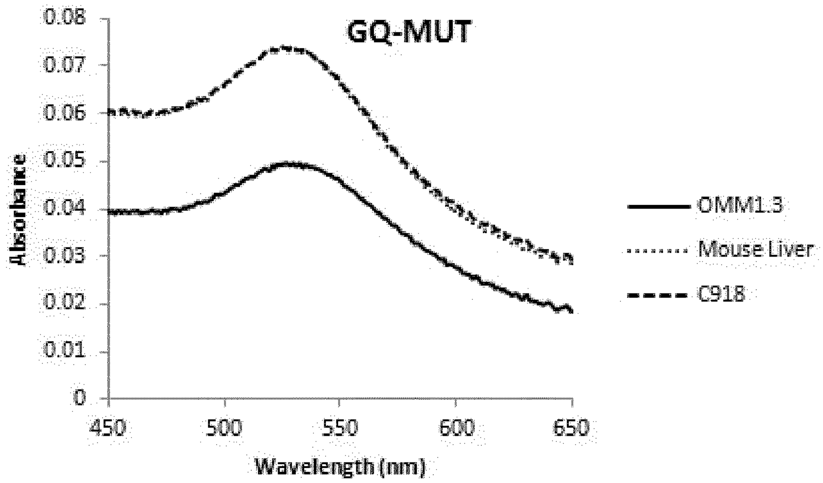
GQ-MUT
Absorbance
0.08
0.07
0.06
0.05
0.04
0.03
0.02
0.01
0
450
500
550
600
650
Wavelength (nm)
OMM1.3
Mouse Liver
C918

Fig. 10

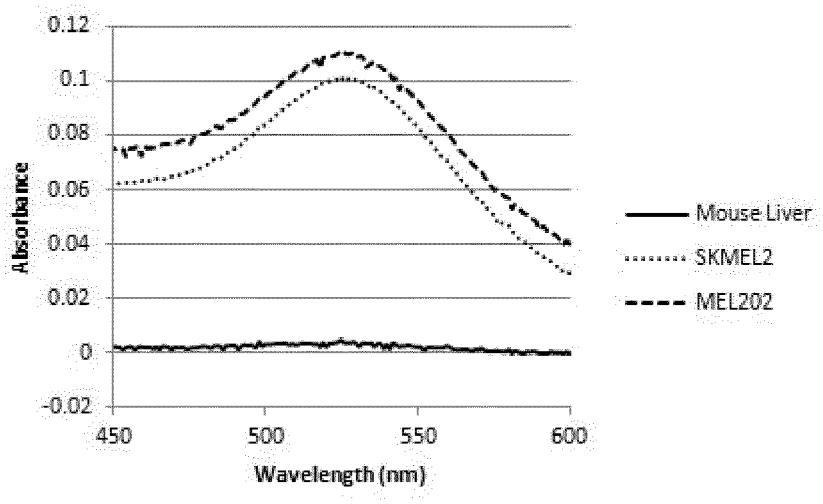
Absorbance
0.12
0.1
0.08
0.06
0.04
0.02
0
-0.02
450
500
550
600
Wavelength (nm)
Mouse Liver
SKMEL2
MEL202

Fig. 11

Europäisches Patentamt
European Patent Office
Office européen des brevets

# EUROPEAN SEARCH REPORT

Application Number
EP 14 38 2033

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br>Y | WO 2007/059514 A2 (OXONICA INC [US]; NATAN MICHAEL J [US]; SHA MICHAEL [US]; DOERING WILL) 24 May 2007 (2007-05-24)<br>* examples; figure 6; [0040] * | 1-3,5, 10-15<br><br>4,6-9 | INV.<br>C12Q1/68 |
| X | DUBERTRET B ET AL: "Single-mismatch detection using gold-quenched fluorescent oligonucleotides",<br>NATURE BIOTECHNOLOGY, NATURE PUBLISHING GROUP, NEW YORK, NY, US,<br>vol. 19, no. 4, 1 April 2001 (2001-04-01), pages 365-370, XP002224627,<br>ISSN: 1087-0156, DOI: 10.1038/86762<br>* figure 1B; page 365, column 2; page 367, column 2 * | 1,10-15 | |
| X,D | WO 02/18951 A2 (UNIV ROCKEFELLER [US]; DUBERTRET BENOIT [US]; CALAME MICHEL [CH]; LIBC) 7 March 2002 (2002-03-07)<br>* Examples; figure 1b * | 1,10-15 | |
| Y | JIANPENG XUE ET AL: "Visual detection of STAT5B gene expression in living cell using the hairpin DNA modified gold nanoparticle beacon",<br>BIOSENSORS AND BIOELECTRONICS,<br>vol. 41, 1 March 2013 (2013-03-01), pages 71-77, XP055123117,<br>ISSN: 0956-5663, DOI: 10.1016/j.bios.2012.06.062<br>* page 72, figure 1; page 73, item 2.2 * | 4,6 | TECHNICAL FIELDS SEARCHED (IPC)<br>C12Q |
| Y | US 2010/201381 A1 (IQBAL SAMIR M [US] ET AL) 12 August 2010 (2010-08-12)<br>* page 11, [0300]; claim 25; claim1 * | 7-9 | |
| | -/-- | | |

1

The present search report has been drawn up for all claims

| Place of search | Date of completion of the search | Examiner |
|---|---|---|
| Munich | 12 June 2014 | Hennard, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 38 2033

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | NOOR MOHAMMUD ET AL: "Electrical detection of single-base DNA mutation using functionalized nanoparticles", APPLIED PHYSICS LETTERS, AMERICAN INSTITUTE OF PHYSICS, US, vol. 95, no. 7, 17 August 2009 (2009-08-17), pages 73703-73703, XP012122768, ISSN: 0003-6951, DOI: 10.1063/1.3152768<br>* Page 95, figure 1 *<br>----- | 1-15 | |
| | | | TECHNICAL FIELDS SEARCHED (IPC) |

The present search report has been drawn up for all claims

1

| Place of search | Date of completion of the search | Examiner |
|---|---|---|
| Munich | 12 June 2014 | Hennard, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 14 38 2033

This annex lists the patent family members relating to the patent documents cited in the above-mentioned European search report.
The members are as contained in the European Patent Office EDP file on
The European Patent Office is in no way liable for these particulars which are merely given for the purpose of information.

12-06-2014

| Patent document cited in search report | Publication date | Patent family member(s) | Publication date |
|---|---|---|---|
| WO 2007059514 A2 | 24-05-2007 | EP 1948829 A2 | 30-07-2008 |
| | | JP 2009523406 A | 25-06-2009 |
| | | US 2009298197 A1 | 03-12-2009 |
| | | WO 2007059514 A2 | 24-05-2007 |
| WO 0218951 A2 | 07-03-2002 | AU 9323201 A | 13-03-2002 |
| | | US 2004002089 A1 | 01-01-2004 |
| | | WO 0218951 A2 | 07-03-2002 |
| US 2010201381 A1 | 12-08-2010 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2002018951 A


### Non-patent literature cited in the description


- **Y. TU et al.** *Chem. Commun.,* 2012, vol. 48, 10718-10720
- Handbook of Chemistry. MARUZEN Co., Ltd, 168-177
- Syntheses and Characterizations: 3.2 Synthesis of Metal Nanoparticles. **BRADLEY, J. S. ; SCHMID, G. ; TALAPIN, D. V. ; SHEVCHENKO, E. V. ; WELLER, H.** Nanoparticles: From Theory to Application. Wiley-VCH Verlag GmbH & Co. KGaA, 2005
- **NARANG et al.** *Meth. Enzymol.,* 1979, vol. 68, 90
- **BROWN et al.** *Meth. Enzymol.,* 1979, vol. 68, 109
- **CHU et al.** *Nucl. Acids. Res.,* 1983, vol. 11, 6513-6529
- **SAMBROOK et al.** Molecular Cloning: A Laboratory Manual. Cold Spring Harbor Laboratory, 1989
- **WETMUR.** *Critical Reviews in Biochem. and Mol. Biol.,* 1991, vol. 26 (3/4), 227-259
- **SAMBROOK et al.** Molecular cloning: a Laboratory Manual. Cold Spring Harbor Laboratory Press, 2001, vol. 1-3
- **ALTSCHUL, S. et al.** *Mol. Biol.,* 1990, vol. 215, 403-410
- **AMBROSI et al.** *Analytical chemistry,* 2007, vol. 79, 5232-40